# EUROPEAN PATENT SPECIFICATION

(11) **EP 1 412 531 B1**
(45) Date of publication and mention of the grant of the patent: **23.04.2008**
(21) Application number: 02762370.1
(22) Date of filing: 17.07.2002
(51) Int. Cl.: C12Q 1/68

(54) **IL-4 RECEPTOR SEQUENCE VARIATION ASSOCIATED WITH TYPE 1 DIABETES**
IL-4 REZEPTOR SEQUENZVARIANTEN, DIE MIT TYP I DIABETES ASSOZIIERT SIND
VARIATION DE LA SEQUENCE DU RECEPTEUR DE L'IL-4 ASSOCIEE AUX DIABETES DE TYPE 1

(30) Priority: 20.07.2001 US 306912 P
(43) Date of publication of application: 28.04.2004
(73) Proprietor: Roche Diagnostics GmbH, 68305 Mannheim (DE); F. Hoffmann-La Roche AG, 4070 Basel (CH)
(72) Inventor: MIREL, Daniel, B., Oakland, CA 94610 (US); ERLICH, Henry, A. Roche Molecular Systems, Inc., Alameda, CA 94501 (US); BUGAWAN, Teodorica, L., Castro Valley, CA 94552 (US); NOBLE, Janelle, A., Berkeley, CA 94702 (US); VALDEZ, Ana, Maria, 40069 Zola Predosa (BO) (IT)
(86) International application number: PCT/EP2002/007956
(87) International publication number: WO 2003/010335

(56) References cited:
- REIMSNIDER SHARON K ET AL: "IL4 and IL4Ralpha genes are not linked or associated with type 1 diabetes." PEDIATRIC RESEARCH, vol. 47, no. 2, February 2000 (2000-02), pages 246-249, XP002243326 ISSN: 0031-3998
- KANEMITSU S ET AL: "Association of interleukin-4 receptor and interleukin-4 promoter gene polymorphisms with systemic lupus erythematosus" ARTHRITIS AND RHEUMATISM, LIPPINCOTT, PHILADELPHIA, US, vol. 42, no. 6, June 1999 (1999-06), pages 1298-99, XP001064873 ISSN: 0004-3591
- HACKSTEIN H ET AL: "Definition of human interleukin-4 receptor alpha chain haplotypes and allelic association with atopy markers." HUMAN IMMUNOLOGY, vol. 60, no. 11, November 1999 (1999-11), pages 1119-1127, XP002243327 ISSN: 0198-8859
- WU X ET AL: "A population genetics study of single nucleotide polymorphisms in the interleukin 4 receptor alpha (IL4RA) gene." GENES AND IMMUNITY. ENGLAND MAY 2001, vol. 2, no. 3, May 2001 (2001-05), pages 128-134, XP009011815 ISSN: 1466-4879
- SHIRAKAWA I ET AL: "Atopy and asthma: genetic variants of IL-4 and IL-13 signalling." IMMUNOLOGY TODAY. ENGLAND FEB 2000, vol. 21, no. 2, February 2000 (2000-02), pages 60-64, XP002243328 ISSN: 0167-5699
- TISCH ROLAND ET AL: "Insulin-dependent diabetes mellitus" CELL, CELL PRESS, CAMBRIDGE, NA, US, vol. 85, no. 3, 1996, pages 291-297, XP002177472 ISSN: 0092-8674
- MIREL DANIEL B ET AL: "Association of IL4R haplotypes with type 1 diabetes." DIABETES. UNITED STATES NOV 2002, vol. 51, no. 11, November 2002 (2002-11), pages 3336-3341, XP002243337 ISSN: 0012-1797

### SEQUENCE LISTING

<110> F. Hoffmann-La Roche AG
<120> IL-4 RECEPTOR SEQUENCE VARIATION ASSOCIATED WITH TYPE 1 DIABETES
<130> case 21245WO
<150> US 60/306912
   <151> 2001-07-20
<160> 62
<170> PatentIn version 3.1
<210> 1
   <211> 3597
   <212> DNA
   <213> Homo sapiens
<400> 1
   ggcgaatgga gcaggggcgc gcagataatt aaagatttac acacagctgg aagaaatcat      60
   agagaagccg ggcgtggtgg ctcatgccta taatcccagc acttttggag gctgaggcgg     120
   gcagatcact tgagatcagg agttcgagac cagcctggtg ccttggcatc tcccaatggg     180
   gtggctttgc tctgggctcc tgttccctgt gagctgcctg gtcctgctgc aggtggcaag     240
   ctctgggaac atgaaggtct tgcaggagcc cacctgcgtc tccgactaca tgagcatctc     300
   tacttgcgag tggaagatga atggtcccac caattgcagc accgagctcc gcctgttgta     360
   ccagctggtt tttctgctct ccgaagccca cacgtgtatc cctgagaaca acggaggcgc     420
   ggggtgcgtg tgccacctgc tcatggatga cgtggtcagt gcggataact atacactgga     480
   cctgtgggct gggcagcagc tgctgtggaa gggctccttc aagcccagcg agcatgtgaa 540
   acccagggcc ccaggaaacc tgacagttca caccaatgtc tccgacactc tgctgctgac 600
   ctggagcaac ccgtatcccc ctgacaatta cctgtataat catctcacct atgcagtcaa 660
   catttggagt gaaaacgacc cggcagattt cagaatctat aacgtgacct acctagaacc 720
   ctccctccgc atcgcagcca gcaccctgaa gtctgggatt tcctacaggg cacgggtgag 780
   ggcctgggct cagtgctata acaccacctg gagtgagtgg agccccagca ccaagtggca 840
   caactcctac agggagccct tcgagcagca cctcctgctg ggcgtcagcg tttcctgcat 900
   tgtcatcctg gccgtctgcc tgttgtgcta tgtcagcatc accaagatta agaaagaatg 960
   gtgggatcag attcccaacc cagcccgcag ccgcctcgtg gctataataa tccaggatgc 1020
   tcaggggtca cagtgggaga agcggtcccg aggccaggaa ccagccaagt gcccacactg 1080
   gaagaattgt cttaccaagc tcttgccctg ttttctggag cacaacatga aaagggatga 1140
   agatcctcac aaggctgcca aagagatgcc tttccagggc tctggaaaat cagcatggtg 1200
   cccagtggag atcagcaaga cagtcctctg gccagagagc atcagcgtgg tgcgatgtgt 1260
   ggagttgttt gaggccccgg tggagtgtga ggaggaggag gaggtagagg aagaaaaagg 1320
   gagcttctgt gcatcgcctg agagcagcag ggatgacttc caggagggaa gggagggcat 1380
   tgtggcccgg ctaacagaga gcctgttcct ggacctgctc ggagaggaga atgggggctt 1440
   ttgccagcag gacatggggg agtcatgcct tcttccacct tcgggaagta cgagtgctca 1500
   catgccctgg gatgagttcc caagtgcagg gcccaaggag gcacctccct ggggcaagga 1560
   gcagcctctc cacctggagc caagtcctcc tgccagcccg acccagagtc cagacaacct 1620
   gacttgcaca gagacgcccc tcgtcatcgc aggcaaccct gcttaccgca gcttcagcaa 1680
   ctccctgagc cagtcaccgt gtcccagaga gctgggtcca gacccactgc tggccagaca 1740
   cctggaggaa gtagaacccg agatgccctg tgtcccccag ctctctgagc caaccactgt 1800
   gccccaacct gagccagaaa cctgggagca gatcctccgc cgaaatgtcc tccagcatgg 1860
   ggcagctgca gcccccgtct cggcccccac cagtggctat caggagtttg tacatgcggt 1920
   ggagcagggt ggcacccagg ccagtgcggt ggtgggcttg ggtcccccag gagaggctgg 1980
   ttacaaggcc ttctcaagcc tgcttgccag cagtgctgtg tccccagaga aatgtgggtt 2040
   tggggctagc agtggggaag aggggtataa gcctttccaa gacctcattc ctggctgccc 2100
   tggggaccct gccccagtcc ctgtcccctt gttcaccttt ggactggaca gggagccacc 2160
   tcgcagtccg cagagctcac atctcccaag cagctcccca gagcacctgg gtctggagcc 2220
   gggggaaaag gtagaggaca tgccaaagcc cccacttccc caggagcagg ccacagaccc 2280
   ccttgtggac agcctgggca gtggcattgt ctactcagcc cttacctgcc acctgtgcgg 2340
   ccacctgaaa cagtgtcatg gccaggagga tggtggccag acccctgtca tggccagtcc 2400
   ttgctgtggc tgctgctgtg gagacaggtc ctcgccccct acaacccccc tgagggcccc 2460
   agacccctct ccaggtgggg ttccactgga ggccagtctg tgtccggcct ccctggcacc 2520
   ctcgggcatc tcagagaaga gtaaatcctc atcatccttc catcctgccc ctggcaatgc 2580
   tcagagctca agccagaccc ccaaaatcgt gaactttgtc tccgtgggac ccacatacat 2640
   gagggtctct taggtgcatg tcctcttgtt gctgagtctg cagatgagga ctagggctta 2700
   tccatgcctg ggaaatgcca cctcctggaa ggcagccagg ctggcagatt tccaaaagac 2760
   ttgaagaacc atggtatgaa ggtgattggc cccactgacg ttggcctaac actgggctgc 2820
   agagactgga ccccgcccag cattgggctg ggctcgccac atcccatgag agtagagggc 2880
   actgggtcgc cgtgccccac ggcaggcccc tgcaggaaaa ctgaggccct tgggcacctc 2940
   gacttgtgaa cgagttgttg gctgctccct ccacagcttc tgcagcagac tgtccctgtt 3000
   gtaactgccc aaggcatgtt ttgcccacca gatcatggcc cacgtggagg cccacctgcc 3060
   tctgtctcac tgaactagaa gccgagccta gaaactaaca cagccatcaa gggaatgact 3120
   tgggcggcct tgggaaatcg atgagaaatt gaacttcagg gagggtggtc attgcctaga 3180
   ggtgctcatt catttaacag agcttcctta ggttgatgct ggaggcagaa tcccggctgt 3240
   caaggggtgt tcagttaagg ggagcaacag aggacatgaa aaattgctat gactaaagca 3300
   gggacaattt gctgccaaac acccatgccc agctgtatgg ctgggggctc ctcgtatgca 3360
   tggaaccccc agaataaata tgctcagcca ccctgtgggc cgggcaatcc agacagcagg 3420
   cataaggcac cagttaccct gcatgttggc ccagacctca ggtgctaggg aaggcgggaa 3480
   ccttgggttg agtaatgctc gtctgtgtgt tttagtttca tcacctgtta tctgtgtttg 3540
   ctgaggagag tggaacagaa ggggtggagt tttgtataaa taaagtttct ttgtctc 3597
<210> 2
   <211> 2478
   <212> DNA
   <213> Homo sapiens
<400> 2
   atggggtggc tttgctctgg gctcctgttc cctgtgagct gcctggtcct gctgcaggtg 60
   gcaagctctg ggaacatgaa ggtcttgcag gagcccacct gcgtctccga ctacatgagc 120
   atctctactt gcgagtggaa gatgaatggt cccaccaatt gcagcaccga gctccgcctg 180
   ttgtaccagc tggtttttct gctctccgaa gcccacacgt gtatccctga gaacaacgga 240
   ggcgcggggt gcgtgtgcca cctgctcatg gatgacgtgg tcagtgcgga taactataca 300
   ctggacctgt gggctgggca gcagctgctg tggaagggct ccttcaagcc cagcgagcat 360
   gtgaaaccca gggccccagg aaacctgaca gttcacacca atgtctccga cactctgctg 420
   ctgacctgga gcaacccgta tccccctgac aattacctgt ataatcatct cacctatgca 480
   gtcaacattt ggagtgaaaa cgacccggca gatttcagaa tctataacgt gacctaccta 540
   gaaccctccc tccgcatcgc agccagcacc ctgaagtctg ggatttccta cagggcacgg 600
   gtgagggcct gggctcagtg ctataacacc acctggagtg agtggagccc cagcaccaag 660
   tggcacaact cctacaggga gcccttcgag cagcacctcc tgctgggcgt cagcgtttcc 720
   tgcattgtca tcctggccgt ctgcctgttg tgctatgtca gcatcaccaa gattaagaaa 780
   gaatggtggg atcagattcc caacccagcc cgcagccgcc tcgtggctat aataatccag 840
   gatgctcagg ggtcacagtg ggagaagcgg tcccgaggcc aggaaccagc caagtgccca 900
   cactggaaga attgtcttac caagctcttg ccctgttttc tggagcacaa catgaaaagg 960
   gatgaagatc ctcacaaggc tgccaaagag atgcctttcc agggctctgg aaaatcagca 1020
   tggtgcccag tggagatcag caagacagtc ctctggccag agagcatcag cgtggtgcga 1080
   tgtgtggagt tgtttgaggc cccggtggag tgtgaggagg aggaggaggt agaggaagaa 1140
   aaagggagct tctgtgcatc gcctgagagc agcagggatg acttccagga gggaagggag 1200
   ggcattgtgg cccggctaac agagagcctg ttcctggacc tgctcggaga ggagaatggg 1260
   ggcttttgcc agcaggacat gggggagtca tgccttcttc caccttcggg aagtacgagt 1320
   gctcacatgc cctgggatga gttcccaagt gcagggccca aggaggcacc tccctggggc 1380
   aaggagcagc ctctccacct ggagccaagt cctcctgcca gcccgaccca gagtccagac 1440
   aacctgactt gcacagagac gcccctcgtc atcgcaggca accctgctta ccgcagcttc 1500
   agcaactccc tgagccagtc accgtgtccc agagagctgg gtccagaccc actgctggcc 1560
   agacacctgg aggaagtaga acccgagatg ccctgtgtcc cccagctctc tgagccaacc 1620
   actgtgcccc aacctgagcc agaaacctgg gagcagatcc tccgccgaaa tgtcctccag 1680
   catggggcag ctgcagcccc cgtctcggcc cccaccagtg gctatcagga gtttgtacat 1740
   gcggtggagc agggtggcac ccaggccagt gcggtggtgg gcttgggtcc cccaggagag 1800
   gctggttaca aggccttctc aagcctgctt gccagcagtg ctgtgtcccc agagaaatgt 1860
   gggtttgggg ctagcagtgg ggaagagggg tataagcctt tccaagacct cattcctggc 1920
   tgccctgggg accctgcccc agtccctgtc cccttgttca cctttggact ggacagggag 1980
   ccacctcgca gtccgcagag ctcacatctc ccaagcagct ccccagagca cctgggtctg 2040
   gagccggggg aaaaggtaga ggacatgcca aagcccccac ttccccagga gcaggccaca 2100
   gacccccttg tggacagcct gggcagtggc attgtctact cagcccttac ctgccacctg 2160
   tgcggccacc tgaaacagtg tcatggccag gaggatggtg gccagacccc tgtcatggcc 2220
   agtccttgct gtggctgctg ctgtggagac aggtcctcgc cccctacaac ccccctgagg 2280
   gccccagacc cctctccagg tggggttcca ctggaggcca gtctgtgtcc ggcctccctg 2340
   gcaccctcgg gcatctcaga gaagagtaaa tcctcatcat ccttccatcc tgcccctggc 2400
   aatgctcaga gctcaagcca gacccccaaa atcgtgaact ttgtctccgt gggacccaca 2460
   tacatgaggg tctcttag 2478
<210> 3
   <211> 21
   <212> DNA
   <213> Artificial Sequence
   <220>
   <223> Description of Artificial Sequence: probe used to identify IL4R polymorphisms
<400> 3
   ccacacgtgt atccctgaga a 21
<210> 4
   <211> 19
   <212> DNA
   <213> Artificial Sequence
   <220>
   <223> Description of Artificial Sequence: probe used to identify IL4R polymorphisms
<400> 4
   tctcagggac acacgtgtg 19
<210> 5
   <211> 22
   <212> DNA
   <213> Artificial Sequence
   <220>
   <223> Description of Artificial Sequence: probe used to identify IL4R polymorphisms
<400> 5
   tggagtgaaa acgacccggc ag 22
<210> 6
   <211> 21
   <212> DNA
   <213> Artificial Sequence
   <220>
   <223> Description of Artificial Sequence: probe used to identify IL4R polymorphisms
<400> 6
   ctgccgggtc attttcgctc c 21
<210> 7
   <211> 21
   <212> DNA
   <213> Artificial Sequence
   <220>
   <223> Description of Artificial Sequence: probe used to identify IL4R polymorphisms
<400> 7
   gagggaaggg agggcattgt g 21
<210> 8
   <211> 19
   <212> DNA
   <213> Artificial Sequence
   <220>
   <223> Description of Artificial Sequence: probe used to identify IL4R polymorphisms
<400> 8
   agggaagggc gggcattgt 19
<210> 9
   <211> 19
   <212> DNA
   <213> Artificial Sequence
   <220>
   <223> Description of Artificial Sequence: probe used to identify IL4R polymorphisms
<400> 9
   ctctccgagc aggtccagg 19
<210> 10
   <211> 21
   <212> DNA
   <213> Artificial Sequence
   <220>
   <223> Description of Artificial Sequence: probe used to identify IL4R polymorphisms
<400> 10
   tcctggacct tctcggagag g 21
<210> 11
   <211> 23
   <212> DNA
   <213> Artificial Sequence
   <220>
   <223> Description of Artificial Sequence: probe used to identify IL4R polymorphisms
<400> 11
   aaggtggaag aaggcatgac tcc 23
<210> 12
   <211> 23
   <212> DNA
   <213> Artificial Sequence
   <220>
   <223> Description of Artificial Sequence: probe used to identify IL4R polymorphisms
<400> 12
   aaggtgggag aagacatgac tcc 23
<210> 13
   <211> 23
   <212> DNA
   <213> Artificial Sequence
   <220>
   <223> Description of Artificial Sequence: probe used to identify IL4R polymorphisms
<400> 13
   ggagtcacgt cttctcctac ctt 23
<210> 14
   <211> 22
   <212> DNA
   <213> Artificial Sequence
   <220>
   <223> Description of Artificial Sequence: probe used to identify IL4R polymorphisms
<400> 14
   tggctcagag agttgctgaa gc 22
<210> 15
   <211> 19
   <212> DNA
   <213> Artificial Sequence
   <220>
   <223> Description of Artificial Sequence: probe used to identify IL4R polymorphisms
<400> 15
   ttcagcaacc ccctgagcc 19
<210> 16
   <211> 20
   <212> DNA
   <213> Artificial Sequence
   <220>
   <223> Description of Artificial Sequence: probe used to identify IL4R polymorphisms
<400> 16
   agtggctatc aggagtttgt 20
<210> 17
   <311> 20
   <212> DNA
   <213> Artificial Sequence
   <220>
   <223> Description of Artificial Sequence: probe used to identify IL4R polymorphisms
<400> 17
   agtggctatc gggagtttgt 20
<210> 18
   <211> 21
   <212> DNA
   <213> Artificial Sequence
   <220>
   <223> Description of Artificial Sequence: probe used to identify IL4R polymorphisms
<400> 18
   ctcttctctg agatgcccga g 21
<210> 19
   <211> 21
   <212> DNA
   <213> Artificial Sequence
   <220>
   <223> Description of Artificial Sequence: probe used to identify IL4R polymorphisms
<400> 19
   ctcgggcatc ccagagaaga g 21
<210> 20
   <211> 8
   <212> DNA
   <213> Artificial Sequence
   <220>
   <223> Description of Artificial Sequence: sequence from affected and non affected individuals
<400> 20
   acagttat 8
<210> 21
   <211> 8
   <212> DNA
   <213> Artificial Sequence
   <220>
   <223> Description of Artificial Sequence: sequence from affected and non affected individuals
<400> 21
   acagttgt 8
<210> 22
   <211> 8
   <212> DNA
   <213> Artificial Sequence
   <220>
   <223> Description of Artificial Sequence: sequence from affected and non affected individuals
<400> 22
   acctccgt 8
<210> 23
   <211> 8
   <212> DNA
   <213> Artificial Sequence
   <220>
   <223> Description of Artificial Sequence: sequence from affected and non affected individuals
<400> 23
   gcagttat 8
<210> 24
   <211> 8
   <212> DNA
   <213> Artificial Sequence
   <220>
   <223> Description of Artificial Sequence: sequence from affected and non affected individuals
<400> 24
   gccgccgt 8
<210> 25
   <211> 19
   <212> DNA
   <213> Artificial Sequence
   <220>
   <223> Description of Artificial Sequence: primer
<400> 25
   cagcccctgt gtctgcaga 19
<210> 26
   <211> 20
   <212> DNA
   <213> Artificial Sequence
   <220>
   <223> Description of Artificial Sequence: primer
<400> 26
   ctgacctgga gcaacccgta 20
<210> 27
   <211> 25
   <212> DNA
   <213> Artificial Sequence
   <220>
   <223> Description of Artificial Sequence: primer
<400> 27
   attgtgtgag gaggaggagg aggta 25
<210> 28
   <211> 17
   <212> DNA
   <213> Artificial Sequence
   <220>
   <223> Description of Artificial Sequence: primer
<400> 28
   ctcgtcatcg caggcaa 17
<210> 29
   <211> 19
   <212> DNA
   <213> Artificial Sequence
   <220>
   <223> Description of Artificial Sequence: primer
<400> 29
   gccgaaatgt cctccagca 19
<210> 30
   <211> 15
   <212> DNA
   <213> Artificial Sequence
   <220>
   <223> Description of Artificial Sequence: primer
<400> 30
   ccggcctccc tggca 15
<210> 31
   <211> 26
   <212> DNA
   <213> Artificial Sequence
   <220>
   <223> Description of Artificial Sequence: primer
<400> 31
   gtccagtgta tagttatccg cactga 26
<210> 32
   <211> 19
   <212> DNA
   <213> Artificial Sequence
   <220>
   <223> Description of Artificial Sequence: primer
<400> 32
   actgggcctc tgctggtca 19
<210> 33
   <211> 22
   <212> DNA
   <213> Artificial Sequence
   <220>
   <223> Description of Artificial Sequence: primer
<400> 33
   gttgggcatg tgagcactcg ta 22
<210> 34
   <211> 18
   <212> DNA
   <213> Artificial Sequence
   <220>
   <223> Description of Artificial Sequence: primer
<400> 34
   agggcatctc gggttcta 18
<210> 35
   <211> 21
   <212> DNA
   <213> Artificial Sequence
   <220>
   <223> Description of Artificial Sequence: primer
<400> 35
   ccacatttct ctggggacac a 21
<210> 36
   <211> 20
   <212> DNA
   <213> Artificial Sequence
   <220>
   <223> Description of Artificial Sequence: primer
<400> 36
   gcagactcag caacaagagg 20
<210> 37
   <211> 21
   <212> DNA
   <213> Artificial Sequence
   <220>
   <223> Description of Artificial Sequence: hybridization probe
<400> 37
   ccacacgtgt atccctgaga a 21
<210> 38
   <211> 22
   <212> DNA
   <213> Artificial Sequence
   <220>
   <223> Description of Artificial Sequence: hybridization probe
<400> 38
   tggagtgaaa acgacccggc ag 22
<210> 39
   <211> 21
   <212> DNA
   <213> Artificial Sequence
   <220>
   <223> Description of Artificial Sequence: hybridization probe
<400> 39
   gagggaaggg agggcattgt g 21
<210> 40
   <211> 19
   <212> DNA
   <213> Artificial Sequence
   <220>
   <223> Description of Artificial Sequence: hybridization probe
<400> 40
   ctctccgagc aggtccagg 19
<210> 41
   <211> 23
   <212> DNA
   <213> Artificial Sequence
   <220>
   <223> Description of Artificial Sequence: hybridization probe
<400> 41
   aaggtggaag aaggcatgac tcc 23
<210> 42
   <211> 23
   <212> DNA
   <213> Artificial Sequence
   <220>
   <223> Description of Artificial Sequence: hybridization probe
<400> 42
   aaggtgggag aagacatgac tcc 23
<210> 43
   <211> 22
   <212> DNA
   <213> Artificial Sequence
   <220> >
   <223> Description of Artificial Sequence: hybridization probe
<400> 43
   tggctcagag agttgctgaa gc 22
<210> 44
   <211> 20
   <212> DNA
   <213> Artificial Sequence
   <220>
   <223> Description of Artificial Sequence: hybridization probe
<400> 44
   agtggctatc aggagtttgt 20
<210> 45
   <211> 21
   <212> DNA
   <213> Artificial Sequence
   <220>
   <223> Description of Artificial Sequence: hybridization probe
<400> 45
   ctcttctctg agatgcccga g 21
<210> 46
   <211> 19
   <212> DNA
   <213> Artificial Sequence
   <220>
   <223> Description of Artificial Sequence: hybridization probe
<400> 46
   tctcagggac acacgtgtg 19
<210> 47
   <211> 21
   <212> DNA
   <213> Artificial Sequence
   <220>
   <223> Description of Artificial Sequence: hybridization probe
<400> 47
   ctgccgggtc attttcgctc c 21
<210> 48
   <211> 19
   <212> DNA
   <213> Artificial Sequence
   <220>
   <223> Description of Artificial Sequence: hybridization probe
<400> 48
   agggaagggc gggcattgt 19
<210> 49
   <211> 21
   <212> DNA
   <213> Artificial Sequence
   <220>
   <223> Description of Artificial Sequence: hybridization probe
<400> 49
   tcctggacct tctcggagag g 21
<210> 50
   <211> 23
   <212> DNA
   <213> Artificial Sequence
   <220>
   <223> Description of Artificial Sequence: hybridization probe
<400> 50
   ggagtcacgt cttctcctac ctt 23
<210> 51
   <211> 19
   <212> DNA
   <213> Artificial Sequence
   <220>
   <223> Description of Artificial Sequence: hybridization probe
<400> 51
   ttcagcaacc ccctgagcc 19
<210> 52
   <211> 20
   <212> DNA
   <213> Artificial Sequence
   <220>
   <223> Description of Artificial Sequence: hybridization probe
<400> 52
   agtggctatc gggagtttgt 20
<210> 53
   <211> 21
   <212> DNA
   <213> Artificial Sequence
   <220>
   <223> Description of Artificial Sequence: hybridization probe
<400> 53
   ctcgggcatc ccagagaaga g 21
<210> 54
   <211> 21
   <212> DNA
   <213> Artificial Sequence
   <220>
   <223> Description of Artificial Sequence: allele specific PCR primer
<400> 54
   ccacatttct ctggggacac a 21
<210> 55
   <211> 25
   <212> DNA
   <213> Artificial Sequence
   <220>
   <223> Description of Artificial Sequence: allele specific PCR primer
<400> 55
   attgtgtgag gaggaggagg aggta 25
<210> 56
   <211> 25
   <212> DNA
   <213> Artificial Sequence
   <220>
   <223> Description of Artificial Sequence: allele specific PCR primer
<400> 56
   attgtgtgag gaggaggagg aggta 25
<210> 57
   <211> 17
   <212> DNA
   <213> Artificial Sequence
   <220>
   <223> Description of Artificial Sequence: allele specific PCR primer
<400> 57
   ttccaggagg gaaggga 17
<210> 58
   <211> 20
   <212> DNA
   <213> Artificial Sequence
   <220>
   <223> Description of Artificial Sequence: allele specific PCR primer
<400> 58
   caccgcatgt acaaactcct 20
<210> 59
   <211> 17
   <212> DNA
   <213> Artificial Sequence
   <220>
   <223> Description of Artificial Sequence: allele specific PCR primer
<400> 59
   ggtgactggc tcaggga 17
<210> 60
   <211> 17
   <212> DNA
   <213> Artificial Sequence
   <220>
   <223> Description of Artificial Sequence: allele specific PCR primer
<400> 60
   ttccaggagg gaagggc 17
<210> 61
   <211> 20
   <212> DNA
   <213> Artificial Sequence
   <220>
   <223> Description of Artificial Sequence: allele specific PCR primer
<400> 61
   caccgcatgt acaaactccc 20
<210> 62
   <211> 17
   <212> DNA
   <213> Artificial Sequence
   <220>
   <223> Description of Artificial Sequence: allele specific PCR primer
<400> 62
   ggtgactggc tcagggg 17

## Description

### FIELD OF THE INVENTION

The present invention relates to the fields of immunology and molecular biology. More specifically, it relates to methods and reagents for detecting nucleotide sequence variability in the IL4 receptor locus that is associated with type 1 diabetes.

### DESCRIPTION OF RELATED ART

The immunological response to an antigen is mediated through the selective differentiation of CD4+ T helper precursor cells (Th0) to T helper type 1 (Th1) or T helper type 2 (Th2) effector cells, with functionally distinct patterns of cytokine (also described as lymphokine) secretion. Th1 cells secrete interleukin 2 (IL-2), IL-12, tumor necrosis factor (TNF), lymphotoxin (LT), and interferon gamma (IFN-g) upon activation, and are primarily responsible for cell-mediated immunity such as delayed-type hypersensitivity. Th2 cells secrete IL-4, IL-5, IL-6, IL-9, and IL-13 upon activation, and are primarily responsible for extracellular defense mechanisms. The role of Th1 and Th2 cells is reviewed in Peltz, 1991, Immunological Reviews 123: 23-35, incorporated herein by reference.

IL4 and IL 13 play a central role in IgE-dependent inflammatory reactions. IL4 induces IgE antibody production by B Cells and further provides a regulatory function in the differentiation of Th0 to Th1 or Th2 effector cells by both promoting differentiation into Th2 cells and inhibiting differentiation into Th1 cells. IL13 also induces IgE antibody production by B Cells.

IL4 and IL13 operate through the IL4 receptor (IL4R), found on both B and T cells, and the IL13R, found on B cells, respectively. The human IL4 receptor (IL4R) is a heterodimer comprising the IL4R α chain and yc chain. The α-chain of the IL4 receptor also serves as the α-chain of the IL13 receptor. IL4 binds to both IL4R and IL13R through the IL4R α-chain and can activate both B and T cells, whereas IL13 binds only to IL13R through the IL13R α1 chain and activate only T cells.

Reimsneider et al. (Pediatric Research, 2000, vol. 47(2), pp.246-249) and Kanemitsu et al. (Arthritis and Rheumatism, 1999, vol. 42(6), pp. 1298-1299) study various gene polymorphisms in the interleukin-4 receptor gene, and conclude that this gene is not linked or associated with type 1 diabetes.

### SUMMARY OF INVENTION

The present invention relates to a newly discovered association, between sequence variants within the IL-4 receptor (IL4R) shown in table 2 and type 1 diabetes. Identification of the allelic sequence variant(s) present provides information that assists in characterizing individuals according to their risk of type 1 diabetes.

Several single-nucleotide polymorphisms within the IL4R gene have been identified and are indicated in Table 2, below. Although several million sequence variants are possible from the SNPs in Table 2, not all of the possible variants have been observed.

In the methods of the invention, the genotype of the IL4R is determined in order to provide information useful for assessing an individual's risk for particular Thl-mediated diseases, in particular, type 1 diabetes. Individuals who have at least one allele statistically associated with type 1 diabetes possess a factor contributing to the risk of a type 1 diabetes. The statistical association of IL4R alleles (sequence variants) is shown in the examples.

As IL4R is but one component of the complex system of genes involved in an immune response, the effect of the IL4R locus is expected to be small. Other factors, such as an individual's HLA genotype, may exert dominating effects which, in some cases, may mask the effect of the IL4R genotype. For example, particular HLA genotypes are known to have a major effect on the likelihood of type 1 diabetes (see Noble et al., 1996, Am. J. Hum. Genet. 59:1134-1148, incorporated herein by reference). The IL4R genotype is likely to be more informative as an indicator of predisposition towards type 1 diabetes among individuals who have HLA genotypes that confer neither increased nor decreased risk. Furthermore, because allele frequencies at other loci relevant to immune system-related diseases differ between populations and, thus, populations exhibit different risks for immune system-related diseases, it is expected that the effect of the IL4R genotype may be of different magnitude in some populations. Although the contribution of the IL4R genotype may be relatively minor by itself, genotyping at the IL4R locus will contribute information that is, nevertheless, useful for a characterization of an individual's predisposition towards type 1 diabetes. The IL4R genotype information may be particularly useful when combined with genotype information from other loci.

The present invention provides preferred methods, reagents, and kits for IL4R genotyping. The genotype can be determined using any method capable of identifying nucleotide variation consisting of single nucleotide polymorphic sites. The particular method used is not a critical aspect of the invention. A number of suitable methods are described below.

In a preferred embodiment of the invention, genotyping is carried out using oligonucleotide probes specific to variant sequences. Preferably, a region of the IL4R gene which encompasses the probe hybridization region is amplified prior to, or concurrent with, the probe hybridization. Probe-based assays for the detection of sequence variants are well known in the art.

Alternatively, genotyping is carried out using allele-specific amplification or extension reactions, wherein allele-specific primers are used which support primer extension only if the targeted variant sequence is present. Typically, an allele-specific primer hybridizes to the IL4R gene such that the 3' terminal nucleotide aligns with a polymorphic position. Allele-specific amplification reactions and allele-specific extension reactions are well known in the art.

### BRIEF DESCRIPTION OF THE FIGURE

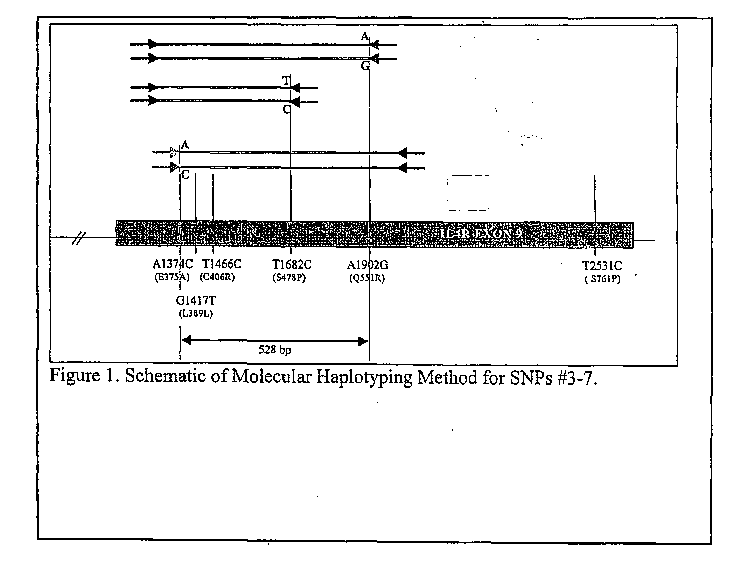

FIG. 1 provides a schematic of a molecular haplotyping method.

### BRIEF DESCRIPTION OF THE TABLES

Table 1 provides the nucleotide sequence of the coding region of an IL4R (SEQ ID NO: 2);

Table 2 provides IL4R SNPs useful in the methods of the invention;

Table 3 provides probes used to identify IL4R polymorphisms (SEQ ID NO: 3-19);

Table 4 provides computationally estimated haplotype frequencies compared between Filipino controls and diabetics (SEQ ID NO: 20-24);

Table 5 provides genotypes of affected and nonaffected individuals;

Table 6 provides single nucleotide polymorphisms detected;

Table 7 provides amplicon primers and lengths (SEQ ID NO: 25-36);

Table 8 provides hybridization probes and titers (SEQ ID NO: 37-53);

Table 9 provides allele frequency of wildtype allele in HBDI founders;

Table 10 provides D' and Δ values for pairs of IL4R SNPs;

Table 11 A provides results of single locus TDT analysis;

Table 11 B provides results of single locus TDT analysis;

Table 12 provides allele-specific PCR primers (SEQ ID NO: 54-62);

Table 13 provides IBD distributions for IL4R haplotypes;

Table 14A provides haplotype transmissions;

Table 14B provides haplotype transmissions;

Table 14C provides haplotype transmissions;

Table 15A provides SNP by SNP allele transmissions;

Table 15B provides SNP by SNP allele transmissions;

Table 16A provides a TDT analysis;

Table 16B provides a TDT analysis;

Table 16C provides a TDT analysis;

Table 17A provides a TDT analysis;

Table 17B provides a TDT analysis;

Table 18 provides allele frequencies in Filipino controls and diabetics;

Table 19 provides estimated haplotype frequencies; and

Table 20 provides observed haplotype frequencies.

### DETAILED DESCRIPTION OF THE INVENTION

To aid in understanding the invention, several terms are defined below.

The term "IL4R gene" refers to the genomic nucleic acid sequence that encodes the interleukin 4 receptor protein. The nucleotide sequence of a gene, as used herein, encompasses coding regions, referred to as exons, intervening, non-coding regions, referred to as introns, and upstream or downstream regions. Upstream or downstream regions can include regions of the gene that are transcribed but not part of an intron or exon, or regions of the gene that comprise, for example, binding sites for factors that modulate gene transcription. The gene sequence of a Human mRNA for IL4R is provided at GenBank accession number X52425 (SEQ ID NO: 1). The coding region is provided as SEQ ID NO: 2.

The term "allele", as used herein, refers to a sequence variant of the gene. Alleles are identified with respect to one or more polymorphic positions, with the rest of the gene sequence unspecified. For example, an IL4R may be defined by the nucleotide present at a single SNP, or by the nucleotides present at a plurality of SNPs. In certain embodiments of the invention, an IL4R is defined by the genotypes of 6, 7 or 8 IL4R SNPs. Examples of such IL4R SNPs are provided in Table 2, below.

For convenience, allele present at the higher or highest frequency in the population will be referred to as the wild-type allele; less frequent allele(s) will be referred to as mutant-allele(s). This designation of an allele as a mutant is meant solely to distinguish the allele from the wild-type allele and is not meant to indicate a change or loss of function.

The term "predisposing allele" refers to an allele that is positively associated with an autoimmune disease such as type 1 diabetes. The presence of a predisposing allele in an individual could be indicative that the individual has an increased risk for the disease relative to an individual without the allele.

The term "protective allele" refers to an allele that is negatively associated with an autoimmune disease such as type 1 diabetes. The presence of a protective allele in an individual could be indicative that the individual has a decreased risk for the disease relative to an individual without the allele.

The terms "polymorphic" and "polymorphism", as used herein, refer to the condition in which two or more variants of a specific genomic sequence, or the encoded amino acid sequence, can be found in a population. The terms refer either to the nucleic acid sequence or the encoded amino acid sequence; the use will be clear from the context. The polymorphic region or polymorphic site refers to a region of the nucleic acid where the nucleotide difference that distinguishes the variants occurs, or, for amino acid sequences, a region of the amino acid where the amino acid difference that distinguishes the protein variants occurs. As used herein, a "single nucleotide polymorphism", or SNP, refers to a polymorphic site consisting of a single nucleotide position.

The term "genotype" refers to a description of the alleles of a gene or genes contained in an individual or a sample. As used herein, no distinction is made between the genotype of an individual and the genotype of a sample originating from the individual. Although, typically, a genotype is determined from samples of diploid cells, a genotype can be determined from a sample of haploid cells, such as a sperm cell.

The haplotype refers to a description of the variants of a gene or genes contained on a single chromosome, i.e, the genotype of a single chromosome.

The term "target region" refers to a region of a nucleic acid which is to be analyzed and usually includes a polymorphic region.

Individual amino acids in a sequence are represented herein as AN or NA, wherein A is the amino acid in the sequence and N is the position in the sequence. In the case that position N is polymorphic, it is convenient to designate the more frequent variant as A₁N and the less frequent variant as NA₂. Alternatively, the polymorphic site, N, is represented as A₁NA₂, wherein A₁ is the amino acid in the more common variant and A₂ is the amino acid in the less common variant. Either the one-letter or three-letter codes are used for designating amino acids (see Lehninger, Biochemistry 2nd ed., 1975, Worth Publishers, Inc. New York, NY: pages 73-75, incorporated herein by reference). For example, 150V represents a single-amino-acid polymorphism at amino acid position 50, wherein isoleucine is the present in the more frequent protein variant in the population and valine is present in the less frequent variant. The amino acid positions are numbered based on the sequence of the mature IL4R protein, as described below.

Representations of nucleotides and single nucleotide changes in DNA sequences are analogous. For example, A398G represents a single nucleotide polymorphism at nucleotide position 398, wherein adenine is the present in the more frequent (wild-type) allele in the population and guanine is present in the less frequent (mutant) allele. The nucleotide positions are numbered based on the IL4R cDNA sequence provided as SEQ ID NO: 2, shown below. It will be clear that in a double stranded form, the complementary strand of each allele will contain the complementary base at the polymorphic position.

Conventional techniques of molecular biology and nucleic acid chemistry, which are within the skill of the art, are fully explained in the literature. See, for example, Sambrook et al., 1989, Molecular Cloning - A Laboratory Manual, Cold Spring Harbor Laboratory, Cold Spring Harbor, New York; Oligonucleotide Synthesis (M.J. Gait, ed., 1984); Nucleic Acid Hybridization (B.D. Hames and S.J. Higgins. eds., 1984); the series, Methods in Enzymology (Academic Press, Inc.); and the series, Current Protocols in Human Genetics (Dracopoli et al., eds., 1984 with quarterly updates, John Wiley & Sons, Inc.); all of which are incorporated herein by reference. All patents, patent applications, and publications mentioned herein, both supra and infra, are incorporated herein by reference.

### METHODS OF THE INVENTION

The present invention provides methods of determining an individual's risk for any autoimmune disease or condition or any Th-1 mediated disease. Such diseases or conditions include, but are not limited to, rheumatoid arthritis, multiple sclerosis, type 1 diabetes mellitus (insulin dependent diabetes mellitus or IDDM), inflammatory bowel diseases, systemic lupus erythematosus, psoriasis, scleroderma, Grave's disease, systemic sclerosis, myasthenia gravis, Gullian-Barre syndromes and Hashimoto's thyroiditis. In certain embodiments of the invention, the methods are used to determine an individual's risk for IDDM. Preferably, the individual is a human.

### IL4R mRNA Sequence

The nucleotide sequence of the coding region of a IL4R mRNA is available from GenBank under accession number X52425, nucleotides 176-2653 and provided as SEQ ID NO: 2, shown in a 5' to 3' orientation in Table 1, below. Although only one strand of the nucleic acid is shown in Table 1, those of skill in the art will recognize that SEQ ID NO: 1 and SEQ ID NO: 2 identify regions of double-stranded genomic nucleic acid, and that the sequences of both strands are fully specified by the sequence information provided.

**Table 1**

| SEQ ID NO: 2 |
|---|
| |

### IL4R SNPs

In the methods of the present invention, the genotype of one or more SNPs in the IL4R gene are determined. The SNPs in the IL4R locus used in the methods of the invention include SNPs in exons, introns or upstream or downstream regions and are provided in Table 2, below, and discussed in detail in the Examples.

In certain embodiments, the genotype of one IL4R SNP can be used to determine an individual's risk for an autoimmune disease. In other embodiments, a the genotypes of a plurality of IL4R SNPs are used. Therefore, the genotypes of 1, 2, 3, 4, 5, 6, 7, 8, 9, 10, 11, 12, 13, 14, 15, 16, 17, 18, 19, 20, 21, 22, 23, 24, 25 or 26 of the SNPs in Table 2 can be used to determine an individual's risk for an autoimmune disease.

**Table 2 IL4R SNPs**

| dbSNP ID rs# | Exon | Variation | **WT allele** | **Var allele** | Acc X52425.1 (cDNA) | AC004525. 1 (genomic) | Formal SNp name |
|---|---|---|---|---|---|---|---|
| | P | C(-3223)T | **G** | **A** | NA | 128387 | G128387A |
| | P | T(-1914)C | **A** | **G** | NA | 127078 | A127078G |
| | 3 | 150V | **A** | **G** | 398 | 94272 | A398G |
| | 4 | N142N | **C** | **T** | 676 | 92548 | C676T |
| | 4 | C92516T | **C** | **T** | Na | 92516 | G92516T |
| | 4 | A92417T | **A** | **T** | Na | 92417 | A92417T |
| 2234896 | 7 | P249P | **C** | **G** | 997 | 80189 | C997G |
| 2234897 | 9 | F288F | **T** | **C** | 1114 | 76868 | T1114C |
| 1805011 | 9 | E375A | **A** | **C** | 1374 | 76608 | A1374C |
| | 9 | E375E | **G** | **A** | 1375 | 76607 | G1375A |
| 2234898 | 9 | L389L | **G** | **T** | 1417 | 76565 | G1417T |
| 1805012 | 9 | C406R | **T** | **C** | 1466 | 76516 | T1466C |
| 2234899 | 9 | C406C | **C** | **T** | 1468 | 76514 | C1468T |
| 2234900 | 9 | L408L | **T** | **C** | 1474 | 76508 | T1474C |
| 1805013 | 9 | S411L | **C** | **T** | 1482 | 76500 | C1482T |
| 1805015 | 9 | S478P | **T** | **C** | 1682 | 76300 | T1682C |
| 1801275 | 9 | Q551R | **A** | **G** | 1902 | 76080 | A1902G |
| | 9 | V5541 | **G** | **A** | 1910 | 76072 | G1910A |
| | 9 | P650S | **C** | **T** | 2198 | 75784 | C2198T |
| 1805016 | 9 | S727A | **T** | **G** | 2429 | 75553 | T2429G |
| | 9 | G759G | **C** | **T** | 2567 | 75455 | C2567T |
| 1805014 | 9 | S761P | **T** | **C** | 2531 | 75451 | T2531C |
| | 9 | P774P | **T** | **C** | 2572 | 75410 | T2572C |
| 1049631 | 9 | 3'UTR | **G** | **A** | 3044 | 74938 | G3044A |
| 8832 | 9 | 3'UTR | **A** | **G** | 3289 | 74693 | A3289G |
| 8674 | 9 | 3'UTR | **C** | **T** | 3391 | 74581 | C3391T |

### Genotyping Methods

In the methods of the present invention, the alleles present in a sample are identified by identifying the nucleotide present at one or more of the polymorphic sites. Any type of tissue containing IL4R nucleic acid may be used for determining the IL4R genotype of an individual. A number of methods are known in the art for identifying the nucleotide present at a single nucleotide polymorphism. The particular method used to identify the genotype is not a critical aspect of the invention. Although considerations of performance, cost, and convenience will make particular methods more desirable than others, it will be clear that any method that can identify the nucleotide present will provide the information needed to identify the genotype. Preferred genotyping methods involve DNA sequencing, allele-specific amplification, or probe-based detection of amplified nucleic acid.

IL4R alleles can be identified by DNA sequencing methods, such as the chain termination method (Sanger et al., 1977, Proc. Natl. Acad. Sci. 74:5463-5467, incorporated herein by reference), which are well known in the art. In one embodiment, a subsequence of the gene encompassing the polymorphic site is amplified and either cloned into a suitable plasmid and then sequenced, or sequenced directly. PCR-based sequencing is described in U.S. Patent No. 5,075,216; Brow, in PCR Protocols, 1990, (Innis et al., eds., Academic Press, San Diego), chapter 24; and Gyllensten, in PCR Technology, 1989 (Erlich, ed., Stockton Press, New York), chapter 5; each incorporated herein by reference. Typically, sequencing is carried out using one of the automated DNA sequencers which are commercially available from, for example, PE Biosystems (Foster City, CA), Pharmacia (Piscataway, NJ), Genomyx Corp. (Foster City, CA), LI-COR Biotech (Lincloln, NE), GeneSys technologies (Sauk City, WI), and Visable Genetics, Inc. (Toronto, Canada).

IL4R alleles can be identified using amplification-based genotyping methods. A number of nucleic acid amplification methods have been described which can be used in assays capable of detecting single base changes in a target nucleic acid. A preferred method is the polymerase chain reaction (PCR), which is now well known in the art, and described in U.S. Patent Nos. 4,683,195; 4,683,202; and 4,965,188; each incorporated herein by reference. Examples of the numerous articles published describing methods and applications of PCR are found in PCR Applications, 1999, (Innis et al., eds., Academic Press, San Diego), PCR Strategies, 1995, (Innis et al., eds., Academic Press, San Diego); PCR Protocols, 1990, (Innis et al., eds., Academic Press, San Diego); and PCR Technology, 1989, (Erlich, ed., Stockton Press, New York); each incorporated herein by reference. Commercial vendors, such as PE Biosystems (Foster City, CA) market PCR reagents and publish PCR protocols.

Other suitable amplification methods include the ligase chain reaction (Wu and Wallace 1988, Genomics 4:560-569); the strand displacement assay (Walker et al., 1992, Proc. Natl. Acad. Sci. USA 89:392-396, Walker et al. 1992, Nucleic Acids Res. 20:1691-1696, and U.S. Patent No. 5,455,166); and several transcription-based amplification systems, including the methods described in U.S. Patent Nos. 5,437,990; 5,409,818; and 5,399,491; the transcription amplification system (TAS) (Kwoh et al., 1989, Proc. Natl. Acad. Sci. USA 86:1173-1177); and self-sustained sequence replication (3SR) (Guatelli et al., 1990, Proc. Natl. Acad. Sci. USA 87:1874-1878 and WO 92/08800); each incorporated herein by reference. Alternatively, methods that amplify the probe to detectable levels can be used, such as Qβ-replicase amplification (Kramer and Lizardi, 1989, Nature 339:401-402, and Lomeli et al., 1989, Clin. Chem. 35:1826-1831, both of which are incorporated herein by reference). A review of known amplification methods is provided in Abramson and Myers, 1993, Current Opinion in Biotechnology 4:41-47, incorporated herein by reference.

Genotyping also can be carried out by detecting IL4R mRNA. Amplification of RNA can be carried out by first reverse-transcribing the target RNA using, for example, a viral reverse transcriptase, and then amplifying the resulting cDNA, or using a combined high-temperature reverse-transcription-polymerase chain reaction (RT-PCR), as described in U.S. Patent Nos. 5,310,652; 5,322,770; 5,561,058; 5,641,864; and 5,693,517; each incorporated herein by reference (see also Myers and Sigua, 1995, in PCR Strategies, supra, chapter 5).

IL4R alleles can be identified using allele-specific amplification or primer extension methods, which are based on the inhibitory effect of a terminal primer mismatch on the ability of a DNA polymerase to extend the primer. To detect an allele sequence using an allele-specific amplification- or extension-based method, a primer complementary to the IL4R gene is chosen such that the 3' terminal nucleotide hybridizes at the polymorphic position. In the presence of the allele to be identified, the primer matches the target sequence at the 3' terminus and primer is extended. In the presence of only the other allele, the primer has a 3' mismatch relative to the target sequence and primer extension is either eliminated or significantly reduced. Allele-specific amplification- or extension-based methods are described in, for example, U.S. Patent Nos. 5,137,806; 5,595,890; 5,639,611; and U.S. Patent No. 4,851,331, each incorporated herein by reference.

Using allele-specific amplification-based genotyping, identification of the alleles requires only detection of the presence or absence of amplified target sequences. Methods for the detection of amplified target sequences are well known in the art. For example, gel electrophoresis (see Sambrook et al., 1989, supra.) and the probe hybridization assays described above have been used widely to detect the presence of nucleic acids.

Allele-specific amplification-based methods of genotyping can facilitate the identification of haplotypes, as described in the examples. Essentially, the allele-specific amplification is used to amplify a region encompassing multiple polymorphic sites from only one of the two alleles in a heterozygous sample. The SNP variants present within the amplified sequence are then identified, such as by probe hybridization or sequencing.

An alternative probe-less method, referred to herein as a kinetic-PCR method, in which the generation of amplified nucleic acid is detected by monitoring the increase in the total amount of double-stranded DNA in the reaction mixture, is described in Higuchi et al., 1992, Bio/Technology 10:413-417; Higuchi et al., 1993, Bio/Technology 11:1026-1030; Higuchi and Watson, in PCR Applications, supra, Chapter 16; U.S. Patent Nos. 5,994,056 and 6,171,785; and European Patent Publication Nos. 487,218 and 512,334, each incorporated herein by reference. The detection of double-stranded target DNA relies on the increased fluorescence that DNA-binding dyes, such as ethidium bromide, exhibit when bound to double-stranded DNA. The increase of double-stranded DNA resulting from the synthesis of target sequences results in an increase in the amount of dye bound to double-stranded DNA and a concomitant detectable increase in fluorescence. For genotyping using the kinetic-PCR methods, amplification reactions are carried out using a pair of primers specific for one of the alleles, such that each amplification can indicate the presence of a particular allele. By carrying out two amplifications, one using primers specific for the wild-type allele and one using primers specific for the mutant allele, the genotype of the sample with respect to that SNP can be determined. Similarly, by carrying out four amplifications, each with one of the possible pairs possible using allele specific primers for both the upstream and downstream primers, the genotype of the sample with respect to two SNPs can be determined. This gives haplotype information for a pair of SNPs.

Alleles can be identified using probe-based methods, which rely on the difference in stability of hybridization duplexes formed between the probe and the IL4R alleles, which differ in the degree of complementarity. Under sufficiently stringent hybridization conditions, stable duplexes are formed only between the probe and the target allele sequence. The presence of stable hybridization duplexes can be detected by any of a number of well known methods. In general, it is preferable to amplify the nucleic acid prior to hybridization in order to facilitate detection. However, this is not necessary if sufficient nucleic acid can be obtained without amplification.

A probe suitable for use in the probe-based methods of the present invention, which contains a hybridizing region either substantially complementary or exactly complementary to a target region of SEQ ID NO: 2 or the complement of SEQ ID NO: 2, wherein the target region encompasses the polymorphic site, and exactly complementary to one of the two allele sequences at the polymorphic site, can be selected using the guidance provided herein and well known in the art. Similarly, suitable hybridization conditions, which depend on the exact size and sequence of the probe, can be selected empirically using the guidance provided herein and well known in the art. The use of oligonucleotide probes to detect single base pair differences in sequence is described in, for example, Conner et al., 1983, Proc. Natl. Acad. Sci. USA 80:278-282, and U.S. Patent Nos. 5,468,613 and 5,604,099, each incorporated herein by reference.

In preferred embodiments of the probe-based methods for determining the IL4R genotype, multiple nucleic acid sequences from the IL4R gene which encompass the polymorphic sites are amplified and hybridized to a set of probes under sufficiently stringent hybridization conditions. The IL4R alleles present are inferred from the pattern of binding of the probes to the amplified target sequences. In this embodiment, amplification is carried out in order to provide sufficient nucleic acid for analysis by probe hybridization. Thus, primers are designed such that regions of the IL4R gene encompassing the polymorphic sites are amplified regardless of the allele present in the sample. Allele-independent amplification is achieved using primers which hybridize to conserved regions of the IL4R gene. The IL4R gene sequence is highly conserved and suitable allele-independent primers can be selected routinely from SEQ ID NO: 1. One of skill will recognize that, typically, experimental optimization of an amplification system is helpful.

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Patent Nos. 5,310,893; 5,451,512; 5,468,613; and 5,604,099; each incorporated herein by reference.

In a dot-blot format, amplified target DNA is immobilized on a solid support, such as a nylon membrane. The membrane-target complex is incubated with labeled probe under suitable hybridization conditions, unhybridized probe is removed by washing under suitably stringent conditions, and the membrane is monitored for the presence of bound probe. A preferred dot-blot detection assay is described in the examples.

In the reverse dot-blot (or line-blot) format, the probes are immobilized on a solid support, such as a nylon membrane or a microtiter plate. The target DNA is labeled, typically during amplification by the incorporation of labeled primers. One or both of the primers can be labeled. The membrane-probe complex is incubated with the labeled amplified target DNA under suitable hybridization conditions, unhybridized target DNA is removed by washing under suitably stringent conditions, and the membrane is monitored for the presence of bound target DNA. A preferred reverse line-blot detection assay is described in the examples.

Probe-based genotyping can be carried out using a "TaqMan" or "5'-nuclease assay", as described in U.S. Patent Nos. 5,210,015; 5,487,972; and 5,804,375; and Holland et al., 1988, Proc. Natl. Acad. Sci. USA 88:7276-7280, each incorporated herein by reference. In the TaqMan assay, labeled detection probes that hybridize within the amplified region are added during the amplification reaction mixture. The probes are modified so as to prevent the probes from acting as primers for DNA synthesis. The amplification is carried out using a DNA polymerase that possesses 5' to 3' exonuclease activity, e.g., *Tth* DNA polymerase. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

Any method suitable for detecting degradation product can be used in the TaqMan assay. In a preferred method, the detection probes are labeled with two fluorescent dyes, one of which is capable of quenching the fluorescence of the other dye. The dyes are attached to the probe, preferably one attached to the 5' terminus and the other is attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Patent Nos. 5,491,063 and 5,571,673, both incorporated herein by reference, describe alternative methods for detecting the degradation of probe which occurs concomitant with amplification.

The TaqMan assay can be used with allele-specific amplification primers such that the probe is used only to detect the presence of amplified product. Such an assay is carried out as described for the kinetic-PCR-based methods described above. Alternatively, the TaqMan assay can be used with a target-specific probe.

The assay formats described above typically utilize labeled oligonucleotides to facilitate detection of the hybrid duplexes. Oligonucleotides can be labeled by incorporating a label detectable by spectroscopic, photochemical, biochemical, immunochemical, or chemical means. Useful labels include 32P, fluorescent dyes, electron-dense reagents, enzymes (as commonly used in ELISAS), biotin, or haptens and proteins for which antisera or monoclonal antibodies are available. Labeled oligonucleotides of the invention can be synthesized and labeled using the techniques described above for synthesizing oligonucleotides. For example, a dot-blot assay can be carried out using probes labeled with biotin, as described in Levenson and Chang, 1989, in PCR Protocols: A Guide to Methods and Applications (Innis et al., eds., Academic Press. San Diego), pages 99-112, incorporated herein by reference. Following hybridization of the immobilized target DNA with the biotinylated probes under sequence-specific conditions, probes which remain bound are detected by first binding the biotin to avidin-horseradish peroxidase (A-HRP) or streptavidin-horseradish peroxidase (SA-HRP), which is then detected by carrying out a reaction in which the HRP catalyzes a color change of a chromogen.

Whatever the method for determining which oligonucleotides of the invention selectively hybridize to IL4R allelic sequences in a sample, the central feature of the typing method involves the identification of the IL4R alleles present in the sample by detecting the variant sequences present. The allelic sequence (target) can be DNA or RNA.

The present invention also relates to kits, container units comprising useful components for practicing the present method. A useful kit can contain oligonucleotide probes specific for the IL4R alleles. In some cases, detection probes may be fixed to an appropriate support membrane. The kit can also contain amplification primers for amplifying a region of the IL4R locus encompassing the polymorphic site, as such primers are useful in the preferred embodiment of the invention. Alternatively, useful kits can contain a set of primers comprising an allele-specific primer for the specific amplification of IL4R alleles. Other optional components of the kits include additional reagents used in the genotyping methods as described herein. For example, a kit additionally can contain an agent to catalyze the synthesis of primer extension products, substrate nucleoside triphosphates, means for labeling and/or detecting nucleic acid (for example, an avidin-enzyme conjugate and enzyme , substrate and chromogen if the label is biotin), appropriate buffers for amplification or hybridization reactions, and instructions for carrying out the present method.

The examples of the present invention presented below are provided only for illustrative purposes and not to limit the scope of the invention. Numerous embodiments of the invention within the scope of the claims that follow the examples will be apparent to those of ordinary skill in the art from reading the foregoing text and following examples.

### Example 1: Genotyping Protocol: Probe-Based Identification of IL4R Alleles

This example describes an genotyping method in which six regions of the IL4R gene that encompass eight polymorphic sites are amplified simultaneously and the nucleotide present at each of the eight sites is identified by probe hybridization. The probe detection is carried out using an immobilized probe (line blot) format.

### Amplification Primers

Amplification of six regions of the IL4R gene, which encompass eight polymorphic sites, is carried out using the primer pairs shown below. All primers are shown in the 5' to 3' orientation.
The following primers amplify a 114 base-pair region encompassing codon 398.

| | | |
|---|---|---|
| RR192B | (SEQ ID NO: 25) | CAGCCCCTGTGTCTGCAGA |
| RR193B | (SEQ ID NO: 31) | GTCCAGTGTATAGTTATCCGCACTGA |

The following primers amplify a 163 base-pair region encompassing codon 676.

| | | |
|---|---|---|
| DBM0177B | (SEQ ID NO: 26) | GTGACCTGGAGGAACCCGTA |
| DBM0178B | (SEQ ID NO: 32) | ACTGGGCCTCTGCTGGTCA |

The following primers amplify a 228 base-pair region encompassing codons 1374, 1417, and 1466.

| | | |
|---|---|---|
| DBM0023B | (SEQ ID NO: 27) | ATTGTGTGAGGAGGAGGAGGAGGTA |
| DBM0022B | (SEQ ID NO: 33) | GTTGGGCATGTGAGCACTCGTA |

The following primers amplify a 129 base-pair region encompassing codon 1682.

| | | |
|---|---|---|
| DBM0097B | (SEQ ID NO: 28) | CTCGTCATCGCAGGCAA |
| DBM0098B | (SEQ ID NO: 34) | AGGGCATCTCGGGTTCTA |

The following primers amplify a 198 base-pair region encompassing codon 1902.

| | | |
|---|---|---|
| RR200B | (SEQ ID NO: 29) | GCCGAAATGTCCTCCAGCA |
| RR178B | (SEQ ID NO: 35) | CCACATTTCTCTGGGGACACA |

The following primers amplify a 177 base-pair region encompassing codon 2531.

| | | |
|---|---|---|
| DBM0112B | (SEQ ID NO: 30) | CCGGCCTCCCTGGCA |
| DBM0071B | (SEQ ID NO: 36) | GCAGACTCAGCAACAAGAGG |

To facilitate detection in the probe detection format described below, the primers are labeled with biotin attached to the 5' phosphate. Reagents for synthesizing oligonucleotides with a biotin label attached to the 5' phosphate are commercially available from Clonetech (Palo Alto, CA) and Glenn Research (Sterling, VA). A preferred reagent is Biotin-ON from Clonetech.

### Amplification

The PCR amplification is carried out in a total reaction volume of 25-100 µl containing the following reagents:
0.2 ng/µl purified human genomic DNA
0.2 mM each primer
800 mM total dNTP (200 mM each dATP, dTTP, dCTP, dGTP)
70 mM KCI
12 mM Tris-HCI, pH 8.3
3 mM MgCl2,
0.25 5 units/µl AmpliTaq Gold^{™} DNA polymerase*
* developed and manufactured by Hoffmann-La Roche and commercially available from Applera (Foster City, CA).

Amplification is carried out in a GeneAmp7 PCR System 9600 thermal cycler (Applera, Foster City, CA), using the specific temperature cycling profile shown below.

| | | |
|---|---|---|
| Pre-reaction incubation: | | 94°C for 12.5 minutes |
| 33 cycles: | denature: | 95°C for 45 seconds |
| | anneal: | 61°C for 30 seconds |
| | extend: | 72°C for 45 seconds |
| Final extension: | | 72°C for 7 minutes |
| Hold: | | 10°C-15°C |

### Detection Probes

Preferred probes used to identify the nucleotides present at the 8 SNPs present in the amplified IL4R nucleic acids are described in Table 3. The probes are shown in the 5' to 3' orientation. Two probes are shown for the detection of T1466; a mixture of the two probes is used.

### Probe Hybridization Assay, Immobilized Probe Format

In the immobilized probe format, the probes are immobilized to a solid support prior to being used in the hybridization. The probe-support complex is immersed in a solution containing denatured amplified nucleic acid (biotin labeled) to allow hybridization to occur. Unbound nucleic acid is removed by washing under stringent hybridization conditions, and nucleic acid remaining bound to the immobilized probes is detected using a chromogenic reaction. The details of the assay are described below.

For use in the immobilized probe detection format, described below, a moiety is attached to the 5' phosphate of the probe to facilitate immobilization on a solid support. Preferably, Bovine Serum Albumen (BSA) is attached to the 5' phosphate essentially as described by Tung et al., 1991, Bioconjugate Chem. 2:464-465, incorporated herein by reference. Alternatively, a poly-T tail is added to the 5' end as described in U.S. Patent No 5,451,512, incorporated herein by reference.

The probes are applied in a linear format to sheets of nylon membrane (e.g., BioDyne™ B nylon filters, Pall Corp., Glen Cove, NY) using a Linear Striper and Multispense2000™ controller (IVEK, N. Springfield, VT). Probe titers are chosen to achieve signal balance between the allelic variants; the titers used are provided in the table of probes, above. Each sheet is cut to strips between 0.35 and 0.5 cm in width. To denature the amplification products, 20 µl of amplification product (based on a 50 µl reaction) are added to 20 µl of denaturation solution (1.6% NaOH) and incubated at room temperature for 10 minutes to complete denaturation.

The denatured amplification product (40 ml) is added to the well of a typing tray containing 3 ml of hybridization buffer (4X SSPE, 0.5% SDS) and the membrane strip. Hybridizations is allowed to proceed for 15 minutes at 55°C in a rotating water bath. Following hybridization, the hybridization solution is aspirated, the strip is rinsed in 3 ml warm wash buffer (2X SSPE, 0.5% SDS) by gently rocking strips back and forth, and the wash buffer is aspirated. Following rinsing, the strips are incubated in 3 ml enzyme conjugate solution (3.3 ml hybridization buffer and 12 mL of strepavidin-horseradish peroxidase (SA-HRP)) in the rotating water bath for 5 minutes at 55°C. Then the strips are rinsed with wash buffer, as above, incubated in wash buffer at 55° for 12 minutes (stringent wash), and finally rinsed with wash buffer again.

Target nucleic acid, now HRP-labeled, which remains bound to the immobilized amplification product are visualized as follows. A color development solution is prepared by mixing 100 ml of citrate buffer (0.1 M Sodium Citrate, pH 5.0), 5 ml 3,3',5,5'-tetramethylbenzidine (TMB) solution (2 mg/ml TMB powder from Fluka, Milwaukee, WI, dissolved in 100% EtOH), and 100 µl of 3% hydrogen peroxide. The strips first are rinsed in 0.1 M sodium citrate (pH 5.0) for 5 minutes, then incubated in the color development solution with gentle agitation for 8 to 10 minutes at room temperature in the dark. The TMB, initially colorless, is converted by the target-bound HRP, in the presence of hydrogen peroxide, into a colored precipitate. The developed strips are rinsed in water for several minutes and immediately photographed.

### Example 2: Association with Type 1 Diabetes

IL4R genotyping was carried out on individuals from 282 Caucasian families ascertained because they contained two offspring affected with type 1 diabetes. The IL4R genotypes of all individuals were determined. IL4R genotyping was carried out using a genotyping method essentially as described in Example 1. In addition to the 564 offspring (2 sibs in each of 282 families) in the affected sib pairs on which ascertainment was based, there were 26 other affected children. There were 270 unaffected offspring among these families.

The family-based samples were provided as purified genomic DNA from the Human Biological Data Interchange (HBDI), which is a repository for cell lines from families affected with type 1 diabetes. All of the HBDI families used in this study are nuclear families with unaffected parents (genetically unrelated) and at least two affected siblings. These samples are described further in Noble et al., 1996, Am. J. Hum. Genet. 59:1134-1148, incorporated herein by reference.

It is known that the HLA genotype can have a significant effect, either increased or decreased depending on the genotype, on the risk for type 1 diabetes. In particular, individuals with the HLA DR genotype DR3-DQB1*0201/DR4-DQB1*0302 (referred to as DR3/DR4 below) appear to be at the highest risk for type 1 diabetes (see Noble et al., 1996, Am. J. Hum. Genet. 59:1134-1148, incorporated herein by reference). These high-risk individuals have about a 1 in 15 chance of being affected with type 1 diabetes. Because of the strong effect of this genotype on the likelihood of type 1 diabetes, the presence of the DR3-DQB1*0201/DR4-DQB1*0302 genotype could mask the contribution from the IL4R allelic variants.

Individuals within these families also were genotyped at the HLA DRB1 and DQB 1 loci. Of the affected sib pairs, both sibs have the DR3/DR4 genotype in 90 families. Neither affected sib has the DR 3/4 genotype in 144 families. Exactly one of the affected pair has the DR 3/4 genotype in the remaining 48 families.

### Example 3: Association with type I Diabetes in Philippine samples

### Subjects

Samples from 183 individuals from the Philippines were genotyped using the reverse lineblot method essentially as described in Example 1. Among the 183 individuals, 89 individuals have type I diabetes and 94 are matched controls.
(Sample 91IDDM not typed)

### Results

The genotypes of the affected and nonaffected individuals are shown in the Table 4 (SEQ ID NO: 20-24). Both the actual numbers and the frequencies are provided for each genotype. The data (Table 5) confirm the presence of an association of IL4R SNP variants with type I diabetes.

### Example 4: Methods of Genotyping

Eight exemplary SNPs in the human IL4R gene are listed in Table 6. Each SNP is described by its position in the reference GenBank accession sequence X52425.1 (SEQ ID NO: 1). For example, SNP 1 is found at position 398 of X52425.1 (SEQ ID NO: 1), where an "A" nucleotide is present. The variant allele at this position has a "G" nucleotide. The SNPs will be referred to by the SNP # in the subsequent text.

The regions of the IL4R gene that encompass the SNPs are amplified and the nucleotide present identified by probe hybridization. The probe detection is carried out using an immobilized probe (line blot) format, to be described.

### Amplicons and primers

The pairs of primers used to amplify the regions encompassing the eight SNPs are listed in Table 7 (SEQ ID NO: 25-36). SNPs numbers 3, 4, and 5 are co-amplified on the same 228 basepair fragment. The primers are modified at the 5' phosphate by conjugation with biotin. Reagents for synthesizing oligonucleotides with a biotin label attached to the 5' phosphate are commercially available from Clontech (Palo Alto, CA) and Glenn Research (Sterling, VA). A preferred reagent is Biotin-ON from Clontech.

### Amplification conditions

The six amplicons are amplified together in a single PCR reaction in a total reaction volume of 25-100 ml containing the following reagents:
0.2 ng/ml purified human genomic DNA
0.2 mM each primer
800 mM total dNTP (200 mM each dATP, dTTP, dCTP, dGTP)
70 mM KCl
12 mM Tris-HCl, pH 8.3
3 mM MgCl₂
0.25 units/ml AmpliTaq Gold^{™} DNA polymerase*
*developed and manufactured by Hoffinann-La Roche and commercially available from PE Biosystems (Foster City, CA).

Amplification is carried out in a GeneAmp 7 PCR System 9600 thermal cycler (PE Biosystems, Foster City, CA), using the specific temperature cycling profile shown below:

| | | |
|---|---|---|
| Pre-reaction incubation: | | 94°C for 12.5 minutes |
| 33 cycles: | Denature: | 95°C, 45 seconds |
| | Anneal: | 61°C, 30 seconds |
| | Extend: | 72°C, 45 seconds |
| Final Extension: | | 72°C, 7 minutes. |
| | Hold: | 10°C-15°C |

### Hybridization probes and conditions

The probes are immobilized to a solid support prior to being used in the hybridization. The probe-support complex is immersed in a solution containing denatured amplified nucleic acid to allow hybridization to occur. Unbound nucleic acid is removed by washing under sequence-specific hybridization conditions, and nucleic acid remaining bound to the immobilized probes is detected. The detection is carried out using the chromogenic substrate TMB.

For use in the immobilized probe detection format, described below, a moiety is attached to the 5' phosphate of the probe to facilitate immobilization on a solid support. Preferably, Bovine Serum Albumin (BSA) is attached to the 5' phosphate essentially as described by Tung et al., 1991, Bioconjugate Chem. 2:464-465, incorporated herein by reference. Alternatively, a poly-T tail is added to the 3' end as described in U.S. Patent No 5,451,512 incorporated herein by reference.

The probes are applied in a linear format to sheets of nylon membrane using a Linear Striper and Multispense2000™ controller (IVEK, N. Springfield, VT). The allele-specific probes and their titers are shown in Table 8. The detection of the wildtype allele of SNP #5 is carried out using a mixture of two probes as listed; this mixture enables the detection of SNP #5 indiscriminately of another nearby SNP (not relevant to this report). The probe titers listed are chosen to achieve signal balance between the allelic variants. Following probe application, each nylon sheet is cut widthwise into strips between 0.35 and 0.55 cm wide.

To denature the amplification products 20 ml of amplification product is added to 20 ml of denaturation solution (1.6% NaOH) and incubated at room temperature. The denatured amplification product (40 ml) is added to the well of a typing tray containing 3 ml of hybridization buffer (3X SSPE, 0.5% SDS) and the membrane strip. Hybridization is allowed to proceed for 15 minutes at 55° C in a rotating water bath. Following hybridization, the hybridization solution is aspirated, the strip rinsed in 3 ml warm wash buffer (1.5X SSPE, 0.5 % SDS) by gently rocking the strips back and forth, and the wash buffer is aspirated. Following rinsing, the strips are incubated in 3 ml enzyme conjugate solution (3.3 ml hybridization buffer and 12 ml of streptavidin-horseradish peroxidase (SA-HRP)) in the rotating water bath for 5 minutes at 55° C. Then the strips are rinsed with wash buffer, as above, incubated in wash buffer at 55° C for 12 minutes (stringent wash), and finally rinsed with wash buffer again.

Target nucleic acids, now HRP-labeled, which remains bound to the immobilized amplification product are visualized as follows. The strips are rinsed in 0.1 M sodium citrate (pH 5.0) for 5 minutes at room temperature, then incubated in the color development solution with gentle agitation for 8-10 minutes at room temperature in the dark. The color development solution is prepared by mixing 100 ml of citrate buffer (0.1 M sodium citrate, pH 5.0), 5 ml 3,3',5,5'-tetramethylbenzidine (TMB) solution (2 mg/ml TMB powder from Fluka (Milwaukee, WI) dissolved in 100% EtOH), and 100 ml of 3% hydrogen peroxide. The TMB, initially colorless, is converted by the target-bound HRP in the presence of hydrogen peroxide into a colored precipitate. The developed strips are rinsed in water for several minutes and immediately photographed.

### Example 5: Association with type I Diabetes in HBDI subjects

### Subjects

IL4R genotyping was carried out on individuals from 282 Caucasian families ascertained because they contained two offspring affected with type I diabetes. The IL4R genotypes of all individuals were determined. IL4R genotyping was carried out using the reverse-line blot method described. In addition to the 564 offspring (two sibs in each of 282 families in the affected sib pairs on which ascertainment was based), there were 26 other affected children. There were 270 unaffected offspring among these families.

The family-based samples were provided as purified genomic DNA from the Human Biological Data Interchange (HBDI), which is a repository for cell lines from families affected with type I diabetes. All of the HBDI families used in this study are nuclear families with unaffected parents and at least two affected siblings. These samples are described further in Noble et al., 1996, Am. J. Hum. Genet. 59:1134-1148, incorporated herein by reference.

### Statistical analysis, methods and algorithms

Since the eight SNPs in IL4R are both physically and genetically very closely linked to each other, the presence of a particular allele at a particular SNP is correlated with the presence of another particular allele at a nearby SNP. This non-random association of two or more SNPs' alleles is known as linkage disequilibrium (LD).

Linkage disequilibrium among the eight IL4R SNPs was assessed using the genotypes of the 282 pairs of parents. These 564 individuals are not related to each other except by marriage. A summary of the calculated frequency of the WT allele for each SNP in this group of 564 individuals (the "HBDI founders") is shown in Table 9.

The calculation of LD can be performed in several ways. We used two complementary methods to assess LD between all pairs of IL4R SNP loci. In the first method, we calculated the values of two distinct but related metrics for LD, namely D and D (Devlin and Risch 1995), using the Maximum Likelihood Estimation algorithm of Hill (Hill 1974). The values for D and D for all pairs of IL4R SNPs are shown in Table 10, in the lower left triangular portion. Both D and D can have values that range between B1 and +1. Values near +1 or B1 suggest strong linkage disequilibrium; values near zero indicate the absence of LD.

A second measure of LD uses a permutation test method implemented in the Arlequin program (L. Excoffier, University of Geneva, CH) (Excoffier and Slatkin 1995; Slatkin and Excoffier 1996). This method maximizes the likelihood ratio statistic (S= -2log (L_{H}*/L_{H})) by permuting alleles and recalculating S over a large number of iterations until S is maximized. These iterations allow the determination of the null distribution of S, and thus the maximum S obtained can be converted into an exact P-value (significance level). These P-values are listed in the upper right triangular portion of Table 10.

Table 10 of pairwise LD shows that there is significant evidence for LD between SNPs 1 and 2, and among (all combinations of) SNPs 3, 4, 5, 6, 7 and 8. SNPs 3 through 8 are known to exist within 1200 basepairs of each other in a single exon (exon 9) of the IL4R gene, and the LD between these SNPs is evidence for very small genetic distances as well.

The Transmission Disequilibrium Test (TDT) of Spielman (Spielman and Ewens 1996; Spielman and Ewens 1998) was performed on the IL4R genotype data for the 282 affected sib pairs (viz., a family structure consisting of the two parents and the two affected children). The TDT was used to test for the association of the individual alleles of the eight IL4R SNPs to type I diabetes. The TDT assesses whether an allele is transmitted from heterozygous parents to their affected children at a frequency that is significantly different than expected by chance. Under the null hypothesis of no association of an allele with disease, a heterozygous parent will transmit or will not transmit an allele with equal frequency to an affected child. The significance of deviation from the null hypothesis can be assessed using the McNemar chi-squared test statistic (= (T-NT)^2/(T+NT), where T is the observed number of transmissions and NT is the observed number of non-transmissions). The significance (P-value) of the McNemar chi-squared test statistic is equal to the Pearson chi-squared statistic with one degree of freedom (Glantz 1997).

The results of the single SNP locus TDT results are shown in tables 10 A and 10 B. The TDT / S-TDT program (version 1.1) of Spielman was used to perform the counting of transmitted and non-transmitted alleles (Spielman, McGinnis et al. 1993; Spielman and Ewens 1998). The table lists the observed transmissions of the wildtype allele at each SNP locus. Since these are biallelic polymorphisms, the transmission counts of the variant allele are equal to the non-transmissions of the wildtype allele.

The counts of transmissions and non-transmissions of alleles to the probands only shown in Table 11A do not quite reach statistical significance, at a= 0.05. However, it is valid to count transmission events to all affected children. However, when the TDT is used in this way (or, for that matter, with more than one child per family), then a significant test statistic is evidence of linkage only, not of association and linkage. Table 11 B shows the TDT analysis when 26 additional affected children are included. The results presented in Table 11B below show that there is a significant deviation from the expected transmission frequencies for alleles of SNPs 3, 4, 5 and 6. Inspection of the "% transmission" values for these SNPs indicates that the wildtype allele is transmitted to affected children at frequencies greater than the expectation of 50%.

The evidence for strong LD among the eight IL4R SNPs suggested to us that we could detect the transmission of the ordered set of alleles from each parent to each affected child in the HBDI cohort. This ordered set of alleles corresponds physically to one of the two parental chromosomes, and is called a haplotype. By inferring the parental haplotypes and their transmission or non-transmission to affected children, we expect to obtain much more statistical information than from alleles alone.

We inferred IL4R haplotypes using a combination of two methods. As the first step, we used the GeneHunter program (Falling Rain Genomics, Palo Alto, CA) (Kruglyak, Daly et al. 1996), as it very rapidly calculates haplotypes from genotype data from pedigrees. We then inspected each HBDI family pedigree individually using the Cyrillic program (Cherwell Scientific Publishing, Palo Alto, CA), to resolve any ambiguous or unsupported haplotype assignments. Unambiguous and non-recombinant haplotypes could be confidently assigned in all but six of the 282 families. The haplotype data for these 276 families were used in subsequent data analysis.

The IL4R gene has the property that many of the SNPs reside within the 3'-most exon (exon 9), whose coding region is approximately 1.5 kb long. We have exploited this to develop a method for directly haplotyping up to five of these exon 9 alleles (viz., SNPs #3-7) without needing parental genotypes. As many of these SNPs direct changes to the amino acid sequence of the IL4R protein, different haplotypes encode different proteins with likely different functions.

Haplotypes, in an individual for which no parental genotypic information is known, can be inferred unambiguously only when at most one of the SNP sites of those is heterozygous. In other cases, the ambiguity must be resolved experimentally.

We use two allele-specific primers with one common primer to perform PCR reactions (using Stoffel Gold^{™} polymerase) to separately amplify the DNA from each chromosome, as shown in Figure 1 below. The alleles on each amplicon are then detected by the same strip hybridization procedure, and the linked alleles called directly. The choice of allele-specific (colored or shaded arrows) and common (black arrows) primers depends on which SNP loci are heterozygous. The primers are modified at the 5' phosphate by conjugation with biotin, and are shown in Table 12 (SEQ ID NO: 54-62).

For each haplotyping assay, two PCR reactions are set up for each DNA to be tested. One reaction contains the common primer and the wildtype allele-specific primer, the other contains the common primer and the variant allele-specific primer. Each PCR reaction is made in a total reaction volume of 50-100 ml containing the following reagents:0.2 ng/ml purified human genomic DNA
0.2 mM each primer
800 mM total dNTP (200 mM each dATP, dTTP, dCTP, dGTP)
10 mM KCl
10 mM Tris-HCl, pH 8.0
2.5 mM MgCl₂
0.12 units/ml Stoffel GoId™ DNA polymerase*
*developed and manufactured by Roche Molecular Systems but not commercially available.

Amplification is carried out in a GeneAmp 7 PCR System 9600 thermal cycler (PE Biosystems, Foster City, CA), using the specific temperature cycling profile shown below:

| | | |
|---|---|---|
| Pre-reaction incubation: | | 94°C for 12.5 minutes |
| 33 cycles: | Denature: | 95°C, 45 seconds |
| | Anneal: | 64°C, 30 seconds |
| | Extend: | 72°C, 45 seconds |
| | Final Extension: | 72°C, 7 minutes. |
| | Hold: | 10°C-15°C |

Following amplification, each PCR product reaction is denatured and separately used for hybridization to the membrane-bound probes as described above.

### Haplotype sharing in affected sibs

Evidence for linkage of IL4R to type 1 diabetes (as opposed to association) can be assessed by the haplotype sharing method. This method assesses the distribution over all families of the number of chromosomes that are identical-by-descent (IBD) between the two affected siblings in each family. For example, if in a family, the father transmits the same one of his two IL4R haplotypes to both children, and the mother transmits the same one of her two IL4R haplotypes to both children, then the children are said to share two chromosomes IBD (or, to be IBD=2). If both parents transmit different IL4R haplotypes to their two children, the children are said to be IBD=0.

Under the null hypothesis of no linkage of IL4R to type 1 diabetes, the proportion of families IBD=0 is 25%, IBD=1 is 50% and IBD=2 is 25%, as expected by random assortment (see Table 13). Evidence for a statistically significant difference from this expectation can be assessed using the chi-square statistic.

Identity-by-descent (IBD) values of parental IL4R haplotypes in the affected sibs could be determined unambiguously in 256 families. In the rest of the families, one or both parents were homozygous and/or the parental source of the child's chromosomes could not be determined. The distribution of IBD is shown in Table 13.

- It is known that the HLA genotype can have a significant effect, either increased or decreased depending on the genotype, on the risk for type 1 diabetes. In particular, individuals with the HLA DR genotype DR3-DQB1*0201/DR4-DQB1*0302 (referred to as DR3/4 below) appear to be at the highest risk for type 1 diabetes (see Noble, Valdes et al., 1996), incorporated herein by reference). These high-risk individuals have about a 1 in 15 chance of being affected with type 1 diabetes. Because of the strong effect of this genotype on the likelihood of type 1 diabetes, the presence of the DR3/4 genotype could mask the contribution of IL4R alleles or haplotypes.

The distribution of IBD in families was stratified into two groups based on the DR3/4 genotype of the children. The first group contains the families in which one or both of the sibs are DR3/4 ("Either/both sib DR3/4", n=119). The second group contains the families where neither child is DR3/4 ("Neither sib DR3/4", n=137). The IBD distribution in these subgroups is shown in Table 13. There was no statistically significant departure from the expected distribution of IBD sharing in the "either/both sib DR3/4" subgroup of families. There is a statistically significant departure from the expected distribution of IBD sharing in the "neither sib DR3/4" subgroup of families (Table 13). This indicates that there is evidence for linkage of the IL4R loci to IDDM in the "neither sib DR3/4" families.

### Association by AFBAC

Association of IL4R haplotypes with type I diabetes was assessed using the AFBAC (Affected Family Based Control) method (Thomson 1995). In essence, two groups of haplotypes, and the haplotype frequencies in the groups, are compared with each other as in a case/control scheme of sampling. These two groups are the case (transmitted) and the control (AFBAC) haplotypes.

The case haplotypes, namely those transmitted to the affected children, are collected and counted as follows. For every pair of siblings, regardless of the status of the parents (homozygote or heterozygote) we count all four transmitted chromosomes. However, the haplotypes in the two siblings in a pair are not independent of each other. The way to make a statistically conservative and valid enumeration is to divide all counts by two.

The control (AFBAC) haplotypes are those that are never transmitted to the affected pair of children (Thomson 1995). The AFBAC haplotypes permit an unbiased estimate of control haplotype frequencies. AFBACs can only be determined from heterozygous parents, and furthermore, only when the parent transmits one haplotype to both children; the other, never-transmitted haplotype is counted in the AFBAC population. The AFBAC population serves as a well-matched set of control haplotypes for the study.

Table 14A shows the comparison of transmitted and AFBAC frequencies for all HBDI haplotypes that were observed at least five times in the complete sample set. Each row represents data on an individual haplotype. However, in all 16 distinct haplotypes were observed in the HBDI data set, although some very rarely. The seven rarest haplotypes are grouped together in the "others" row. Each haplotype is listed by the allele present at each of the nine IL4R SNPs.

Tables 13B and 13C show the comparison of transmitted and AFBAC frequencies for all HBDI haplotypes seen in the "either/both sib DR3/4" and the "neither sib DR3/4" subgroups of families, respectively. These tables show that stratifying the families based on the DR3/4 genotype of the children permits the identification of haplotypes that are associated with IDDM. In particular, in the "neither sib DR3/4" subgroup one haplotype (labeled "2 1 2 2 2 2 2 1 ") is significantly underrepresented in the pool of transmitted chromosomes (P < 0.005).

From the transmitted and AFBAC haplotype frequency information in Tables 14B and 14C, one can derive by counting the frequencies of transmitted and AFBAC alleles. The locus-by-locus AFBAC analyses are shown in Tables 15A and 15B.

The data present in Tables 15A and 15B show that there is statistically significant evidence, in the "neither sib DR3/4" subgroup of families, that alleles of SNPs numbers 3, 4 5, 6, and 7 are associated with IDDM. The evidence for association is especially strong for SNP #6. In the "either/both sib DR3/4" subgroup, there is the same trend of allelic association, although the trend does not quite reach statistical significance.

### Association by Haplotype-based TDT

The TDT analysis can be utilized for determining the transmission (or non-transmission) of 8-locus haplotypes from parents to affected children, once the haplotypes have been inferred or assigned by molecular means. Tables 16A, B, and C summarize the TDT results for the HBDI families. Table 16A counts informative transmission events only to one child (the proband) per family, Table 16B counts informative transmissions to the two primary affected children per family, and Table 16C counts informative transmissions to all affected children. The 8-locus haplotype TDT results reach statistical significance when all affected children (2 or more per family) are included.

The TDT analyses can be performed on families after stratifying for the DR3 /4 genotype of the children. The summary of counts of informative transmissions to the two primary affected children per family, in the "either/both sib DR3/4" and the "neither sib DR3/4" subgroups of families, are shown in tables 17A and 17B respectively. As presented above, there is significant evidence of linkage of IDDM to IL4R in the "neither sib DR3/4" subgroup. The data in Table 17B indicate that there is significant evidence of association of IL4R haplotypes to IDDM, in the presence of this linkage. In particular, in the "neither sib DR3/4" subgroup one haplotype (labeled "2 1 2 2 2 2 2 1") is significantly under-transmitted to affected children.

### Example 6: Association with type I Diabetes in Philippine samples

Samples from 183 individuals from the Philippines were genotyped using the reverse lineblot method. 89 individuals have type I diabetes, 94 are matched controls.

### Genotyping methods

These subjects were genotyped by the same methods as described above for the HBDI samples. Molecular haplotyping of IL4R SNPs was also performed as described above.

### Statistical methods & algorithms

Allele and haplotype frequencies between groups were compared using the z-test. Haplotype compositions and frequencies were estimated from the genotype data using the Arlequin program (L. Excoffier, University of Geneva, CH) (Excoffier and Slatkin 1995, Slatkin and Excoffier 1996).

### Results

The wildtype allele frequencies for each of the eight IL4R SNPs in the Filipino control and diabetic groups are shown in Table 18. Table 18 provides evidence that the allele frequencies for SNPs #3 and 4 are significantly different between the two groups, and suggests an association to IDDM.

It is also possible to infer and construct the multi-locus IL4R haplotypes in the Filipino subjects, either computationally by Maximum-likelihood estimation (MLE), or by using molecular haplotyping methods described previously. Table 19 lists the five most frequent computationally estimated haplotypes and their frequencies in the Filipino diabetics and controls, and presents the significance of the differences in frequencies.

Table 20 lists the observed haplotypes as derived and inferred by molecular haplotyping; the unambiguous seven-locus haplotypes (SNP#1 allele not shown, as indicated by the "x") are compiled. Tables 18 and 19 both provide evidence of a statistically significant difference in the frequency of one or more haplotypes between the Filipino control and diabetic populations, and support the presence of an association of IL4R to IDDM. In particular, the haplotype (labeled "x 1 2 2 2 2 2 1 ") is significantly underrepresented in the Filipino diabetics group.

### Citations

Devlin, B. and N. Risch (1995). "A comparison of linkage disequilibrium measures for fine-scale mapping." Genomics 29(2): 311-22.
Excoffier, L. and M. Slatkin (1995). "Maximum-likelihood estimation of molecular haplotype frequencies in a diploid population." Mol Biol Evol 12(5): 921-7.
Glantz, S. A. (1997). Primer of biostatistics. New York, McGraw-Hill Health Professions Division.
Hill, W. G. (1974). "Estimation of linkage disequilibrium in randomly mating populations." Heredity 33(2): 229-39.
Kruglyak, L., M. J. Daly, et al. (1996). "Parametric and nonparametric linkage analysis: a unified multipoint approach." Am J Hum Genet 58(6): 1347-63.
Noble, J. A., A. M. Valdes, et al. (1996). "The role of HLA class II genes in insulin-dependent diabetes mellitus: molecular analysis of 180 Caucasian, multiplex families." Am J Hum Genet 59(5): 1134-48.
Slatkin, M. and L. Excoffier (1996). "Testing for linkage disequilibrium in genotypic data using the Expectation-Maximization algorithm." Heredity 76(Pt 4): 377-83.
Spielman, R. S. and W. J. Ewens (1996). "The TDT and other family-based tests for linkage disequilibrium and association." Am J Hum Genet 59(5): 983-9.
Spielman, R. S. and W. J. Ewens (1998). "A sibship test for linkage in the presence of association: the sib transmission/disequilibrium test." Am J Hum Genet 62(2): 450-8.
Spielman, R. S., R. E. McGinnis, et al. (1993). "Transmission test for linkage disequilibrium: the insulin gene region and insulin-dependent diabetes mellitus (IDDM)." Am J Hum Genet **52**(3): 506-16.
Thomson, G. (1995). "Mapping disease genes: family-based association studies." Am J Hum Genet **57**(2): 487-98.

**TABLE 3**

| **Variant** | **Probe** | **Probe Sequence** | **Seq ID No:** | **titer (µl)** |
|---|---|---|---|---|
| A398 | DBM0081P | CCACACGTGTATCCCTGAGAA | 3 | 1.0 |
| 398G | DBM0082P | TCTCAGGGACACACGTGTG | 4 | 4.0 |
| C676 | DBM0172P | TGGAGTGAAAACGACCCGGCAG | 5 | 1.0 |
| 676T | DBM0073P | CTGCCGGGTCATTTTCGCTCC | 6 | 1.0 |
| A1374 | DBM0043RC | GAGGGAAGGGAGGGCATTGTG | 7 | 1.0 |
| 1374C | DBM0139P | AGGGAAGGGCGGGCATTGT | 8 | 4.0 |
| G1417 | DBM0124P | CTCTCCGAGCAGGTCCAGG | 9 | 1.0 |
| 1417T | DBM0046RC | TCCTGGACCTTCTCGGAGAGG | 10 | 1.0 |
| T1466 | DBM0076P | AAGGTGGAAGAAGGCATGACTCC | 11 | 1.0 |
| | DBM0132P | AAGGTGGGAGAAGACATGACTCC | 12 | 1.0 |
| 1466C | DBM0171P | GGAGTCACGTCTTCTCCTACCTT | 13 | 2.0 |
| T1682 | DBM0157P | TGGCTCAGAGAGTTGCTGAAGC | 14 | 2.0 |
| 1682C | DBM0094P | TTCAGCAACCCCCTGAGCC | 15 | 2.0 |
| A1902 | KW 86 | AGTGGCTATCAGGAGTTTGT | 16 | 1.6 |
| 1902G | KW85 | AGTGGCTATCGGGAGTTTGT | 17 | 0.8 |
| T2531 | DBM0080P. | CTCTTCTCTGAGATGCCCGAG | 18 | 0.5 |
| 2531C | DBM0048RC | CTCGGGCATCCCAGAGAAGAG | 19 | 0.5 |

| | **Seq ID No:** | | **MLE frequency** | **MLE frequency** | **O.R.** | **COUNT : control** | **COUNT: diabetics** | **Total count** | **exp control** | **exp diabetic** | **chi-square** | **P-value** |
|---|---|---|---|---|---|---|---|---|---|---|---|---|
| haplotype | | label | | | | | | | | | | |
| ACATTTAT | 20 | H-1 | 0.318 | 0.329 | 1.1 | 59.7 | 58.5 | 118.2 | 60.7 | 57.5 | 0.033 | 0.855 |
| ACAGTTGT | 21 | H-6 | 0.074 | 0.074 | 1.0 | 14.0 | 13.2 | 27.2 | 14.0 | 13.2 | 0.000 | 0.987 |
| ACCTCCGT | 22 | H-3 | 0.145 | 0.058 | 0.4 | 27.3 | 10.3 | 37.6 | 19.3 | 18.3 | 6.727 | 0.009 |
| GCAGTTAT | 23 | H-2 | 0.390 | 0.441 | 1.2 | 73.3 | 78.5 | 151.8 | 78.0 | 73.8 | 0.582 | 0.445 |
| GCCGCCGT | 24 | H-10 | 0.032 | 0.051 | 1.6 | 6.0 | 9.0 | 15.0 | 7.7 | 7.3 | 0.776 | 0.378 |
| | | n = | 188 | 178 | | | | 366 | | | | |
| | | | | | | | | | | overall | 8.118 | 0.0987 |
| Table 4: Computationally estimated haplotype frequencies compared between Filipino controls and diabetics. | | | | | | | | | | | | |

**TABLE 5**

| **SNP** | **Affected Genotypes (n=89)** | | | **Control Genotypes (n=94)** | | |
|---|---|---|---|---|---|---|
| **A398G** | AA=21 | AG=40 | GG=28 | AA=32 | AG=41 | GG=21 |
| **(50 I/V)** | **(0.236)** | **(0.449)** | **(0.315)** | **(0.340)** | **(0.436)** | **(0.223)** |
| **C676G** | CC=89 | | | CC=92 | CG=2 | |
| **(142 N/N)** | **(1)** | | | **(0.979)** | **(0.021)** | |
| **A1374C** | AA=70 | AC=17 | CC=2 | AA=55 | AC=30 | CC=3 |
| **(375 E/A)** | **(0.787)** | **(0.191)** | **(0.023)** | **(0.630)** | **(0.341)** | **(0.034)** |
| **G1417T** | GG=78 | GT=10 | TT=1 | GG=63 | GT=29 | TT=2 |
| **(389 L/L)** | **(0.876)** | **(0.112)** | **(0.011)** | **(0.670)** | **(0.309)** | **(0.022)** |
| **T1466C** | TT=70 | TC=17 | CC=2 | TT=60 | TC=32 | CC=2. |
| **(406 C/R)** | **(0.787)** | **(0.191)** | **(0.023)** | **(0.638)** | **(0.340)** | **(0.022)** |
| **T1682C** | TT=70 | TC=17 | CC=2 | TT=61 | TC=31 | CC=2 |
| **(478 S/P)** | **(0.787)** | **(0.191)** | **(0.023)** | **(0.649)** | **(0.330)** | **(0.022)** |
| **A1902G** | AA=50 | AG=35 | GG=3 | AA=50 | AG=36 | GG=8 |
| **(551 Q/R)** | **(0.562)** | **(0.393)** | **(0.034)** | **(0.532)** | **(0.383)** | **(0.085)** |
| T2531C | TT=89 | | | TT=94 | | |
| **(761 S/P)** | **(1)** | | | **(1)** | | |

| **SNP #** | **LOCUS** | **SNP** | **Variation** | **Genbank Access #** |
|---|---|---|---|---|
| 1 | IL4R | A398G | I50V | X52425 |
| 2 | IL4R | C676T | N142N | X52425 |
| 3 | IL4R | A1374C | E375A | X52425 |
| 4 | IL4R | G1417T | L389L | X52425 |
| 5 | IL4R | T1466C | C406R | X52425 |
| 6 | IL4R | T1682C | S478P | X52425 |
| 7 | IL4R | A1902G | Q551R | X52425 |
| 8 | IL4R | T2531C | S761P | X52425 |
| **Table 6: SNPs detected** | | | | |

| **SNP #** | **Amplicon size, bp** | **Amplicon left Primer Name** | **Left primer sequence** | **Seq ID No:** | **Amplicon Right Primer Name** | **Right primer sequence** | **Seq ID No:** |
|---|---|---|---|---|---|---|---|
| 1 | 114 | RR192B | CAGCCCCTGTGTCTGCAGA | 25 | RR193B | GTCCAGTGTATAGTTATCCGCACTGA | 31 |
| 2 | 163 | DBM0177B | CTGACCTGGAGCAACCCGTA | 26 | DBM0178B | ACTGGGCCTCTGCTGGTCA | 32 |
| 3,4,5 | 228 | DBM0023B | ATTGTGTGAGGAGGAGGAGGAGGTA | 27 | DBM0022B | GTTGGGCATGTGAGCACTCGTA | 33 |
| 6 | 129 | DBM0097B | CTCGTCATCGCAGGCAA | 28 | DBM0098B | AGGGCATCTCGGGTTCTA | 34 |
| 7 | 198 | RR200B | GCCGAAATGTCCTCCAGCA | 29 | RR178B | CCACATTTCTCTGGGGACACA | 35 |
| 8 | 177 | DBM0112B | CCGGCCTCCCTGGCA | 30 | DBM0071B | GCAGACTCAGCAACAAGAGG | 36 |
| Table 7: Amplicon primers and lengths | | | | | | | |

| **SNP #** | **WT allele probe name** | **WT allele probe sequence** | **Seq ID No:** | **WT allele probe titer (µM)** | **Variant allele probe name** | **Variant allele probe sequence** | **Seq ID No:** | **Variant allele probe titer (µM)** |
|---|---|---|---|---|---|---|---|---|
| 1 | DBM0081P | CCACACGTGTATCCCTGAGAA | 37 | 1.0 | DBM0082P | TCTCAGGGACACACGTGTG | 46 | 4.0 |
| 2 | DBM0172P | TGGAGTGAAAACGACCCGGCAG | 38 | 1.0 | DBM0073P | CTGCCGGGTCATTTTCGCTCC | 47 | 1.0 |
| 3 | DBM0043RC | GAGGGAAGGGAGGGCATTGTG | 39 | 1.0 | DBM0139P | AGGGAAGGGCGGGCATTGT | 48 | 4.0 |
| 4 | DBM0124P | CTCTCCGAGCAGGTCCAGG | 40 | 1.0 | DBM0046RC | TCCTGGACCTTCTCGGAGAGG | 49 | 1.0 |
| 5 | DBM0076P + DBM0132P | AAGGTGGAAGAAGGCATGACTCC | 41 | 1.0 | DBM0171P | GGAGTCACGTCTTCTCCTACCTT | 50 | 2.0 |
| | | AAGGTGGGAGAAGACATGACTCC | 42 | 1.0 | | | | |
| 6 | DBM0157P | TGGCTCAGAGAGTTGCTGAAGC | 43 | 2.0 | DBM0094P | TTCAGCAACCCCCTGAGCC | 51 | 2.0 |
| 7 | KW86 | AGTGGCTATCAGGAGTTTGT | 44 | 1.6 | KW85 | AGTGGCTATCGGGAGTTTGT | 52 | 0.8 |
| 8 | DBM0080P. | CTCTTCTCTGAGATGCCCGAG | 45 | 0.5 | DBM0048RC | CTCGGGCATCCCAGAGAAGAG | 53 | 0.5 |
| Table 8: Hybridization probes and titers | | | | | | | | |

| **SNP #** | **WT Allele Frequency** |
|---|---|
| 1 | 0.53 |
| 2 | 0.90 |
| 3 | 0.89 |
| 4 | 0.89 |
| 5 | 0.89 |
| 6 | 0.83 |
| 7 | 0.79 |
| 8 | 0.99 |
| Table 9: Allele Frequency Of wildtype allele in HBDI founders | |

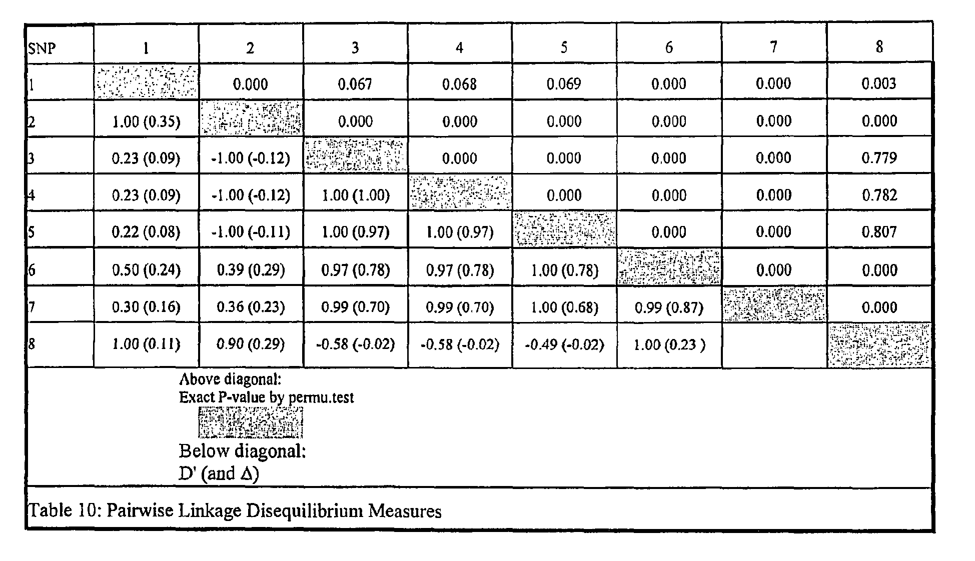

| SNP # | Transmissions of WT allele to affected (T) | Non-transmissions of WT allele to affected (NT) | Total observed Transmissions (T + NT) | McNemar chi- squared statistic | Significance |
|---|---|---|---|---|---|
| 1 | 144 | 138 | 282 | 0.128 | N.S. |
| 2 | 55 | 39 | 94 | 2.723 | N.S. |
| 3 | 62 | 46 | 108 | 2.370 | N.S. |
| 4 | 62 | 46 | 108 | 2.370 | N.S. |
| 5 | 61 | 46 | 107 | 2.103 | N.S. |
| 6 | 90 | 67 | 157 | 3.369 | N.S. |
| 7 | 103 | 81 | 184 | 2.630 | N.S. |
| 8 | 5 | 7 | 12 | 0.333 | N.S. |
| Table 11A: Results of single locus TDT analysis, to the 282 probands only | | | | | |

| SNP # | Transmissions of WT allele to affected (T) | Non-transmissions of WT allele to affected (NT) | Total observed Transmissions + NT) | McNemar (T chi-squared statistic | Significance | % Transmission T / (T + NT) |
|---|---|---|---|---|---|---|
| 1 | 311 | 280 | 591 | 1.626 | N.S. | |
| 2 | 107 | 90 | 197 | 1.467 | N.S. | |
| 3 | 130 | 98 | 228 | 4.491 | P<0.05 | 57.0% |
| 4 | 130 | 98 | 228 | 4.491 | P<0.05 | 57.0% |
| 5 | 128 | 98 | 226 | 3.982 | P<0.05 | 56.6% |
| 6 | 189 | 149 | 338 | 4.734 | P<0.05 | 55.9% |
| 7 | 212 | 180 | 392 | 2.612 | N.S. | |
| 8 | 12 | 13 | 25 | 0.040 | N.S. | |
| Table 11B: Results of single locus TDT analysis, to 564 primary affected and 26 additional children | | | | | | |

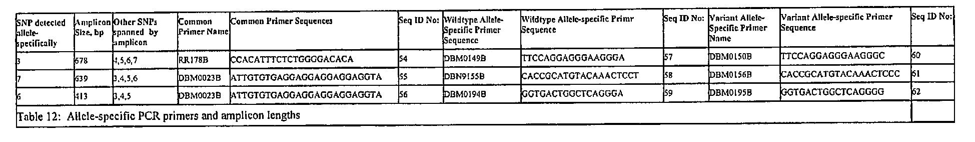

| Group (n) | IBD=2 | IBD=1 | IBD=0 | P-value |
|---|---|---|---|---|
| All families (256) | 30.7% | 47.9% | 21.5% | N.S. |
| Either/both sib DR3/4 (119) | 26.1% | 50.4% | 23.5% | N.S. |
| Neither sib DR3/4 (137) | 34.7% | 45.6% | 19.7% | 0.03 |
| *Expectation* | *25%* | *50%* | *25%* | |
| Table 13: IBD Distributions for IL4R haplotypes | | | | |

| | Counts | | | Observed Frequency | | Chi-squared Statistic | p-value |
|---|---|---|---|---|---|---|---|
| IL4R 8-locus Haplotype | Transmitted | AFBAC | Total | Transmitted | AFBAC | | |
| 1 1 1 1 1 1 1 1 | 259.5 | 109.5 | 369 | 46.3% | 41.8% | 0.82 | 0.36 |
| 2 1 1 1 1 1 1 1 | 170.5 | 71.5 | 242 | 30.4% | 27.3% | 0.60 | 0.44 |
| 2 1 2 2 2 2 2 1 | 29.5 | 23.5 | 53 | 5.3% | 9.0% | 3.79 | 0.05 |
| 2 2 1 1 1 1 1 1 | 23.5 | 13 | 36.5 | 4.2% | 5.0% | 0.24 | 0.63 |
| 1 1 2 2 2 2 2 1 | 23 | 12.5 | 35.5 | 4.1% | 4.8% | 0.18 | 0.67 |
| 2 2 1 1 1 2 2 1 | 20.5 | 14 | 34.5 | 3.7 % | 5.3 % | 1.20 | 0.27 |
| 1 1 1 1 1 1 2 1 | 19 | 9 | 28 | 3.4% | 3.4% | 0.00 | 0.98 |
| 2 1 1 1 1 1 2 1 | 3.5 | 3 | 6.5 | 0.6% | 1.1% | 0.61 | 0.43 |
| 2 2 1 1 1 2 2 2 | 4.5 | 2 | 6.5 | 0.8% | 0.8% | 0.00 | 0.95 |
| Others | 6.5 | 4 | 10.5 | 1.2% | 1.5% | 0.19 | 0.67 |
| TOTAL | 560 | 262 | 822 | | | 7.64 | 0.94 |
| Table 14A: Haplotype transmissions by AFBAC method: all families | | | | | | | |

| | Counts | | | Observed Frequency | | Chi-squared Statistic | p-value |
|---|---|---|---|---|---|---|---|
| IL4R 8-locus Haplotype | Transmitted | AFBAC | Total | Transmitted | AFBAC | | |
| 1 1 1 1 1 1 1 1 | 254 | 50.5 | 304.5 | 46.7% | 41.6% | 0.57 | 0.45 |
| 2 1 1 1 1 1 1 1 | 156 | 30 | 186 | 28.7% | 24.7% | 0.56 | 0.45 |
| 2 1 2 2 2 2 2 1 | 35 | 11 | 46 | 6.4% | 9.1% | 0.99 | 0.32 |
| 2 2 1 1 1 1 1 1 | 24 | 7 | 31 | 4.4% | 5.8% | 0.39 | 0.53 |
| 1 1 1 1 1 1 2 1 | 20 | 6 | 26 | 3.7% | 4.9% | 0.40 | 0.52 |
| 1 1 2 2 2 2 2 1 | 18 | 7 | 25 | 3.3% | 5.8% | 1.59 | 0.21 |
| 2 2 1 1 1 2 2 1 | 18 | 4 | 22 | 3.3% | 3.3% | 0.00 | 0.99 |
| 2 2 1 1 1 2 2 2 | 8 | 2 | 10 | 1.5% | 1.6% | 0.02 | 0.89 |
| 2 21 1 1 2 2 2 | 8 | 1 | 9 | 1.5% | 0.8% | 0.31 | 0.58 |
| 2 1 1 1 1 1 2 1 | 1 | 2 | 3 | 0.2% | 1.6% | 4.71 | 0.03 |
| 2 1 1 1 1 2 2 1 | 2 | 1 | 3 | 0.4% | 0.8% | 0.46 | 0.50 |
| TOTAL | 544 | 121.5 | 665.5 | | | 10.00 | 0.44 |
| Table 14B: Haplotype transmissions by AFBAC method: AEitherlboth sib DR3/4@ subgroup of familes | | | | | | | |

| | Counts | | | Observed Frequency | | Chi-squared Statistic | p-value |
|---|---|---|---|---|---|---|---|
| IL4R 8-locus Haplotype | Transmitted | AFBAC | Total | Transmitted | AFBAC | | |
| 1 1 1 1 1 1 1 1 | 267 | 62.5 | 329.5 | 45.7% | 40.7% | 0.68 | 0.409 |
| 2 1 1 1 1 1 1 1 | 185 | 43 | 228 | 31.7% | 28.0% | 0.53 | 0.467 |
| 2 1 2 2 2 2 2 1 | 24 | 15.5 | 39.5 | 4.1% | 10.1% | 8.14 | 0.004 |
| 1 1 2 2 2 2 2 1 | 30 | 6 | 36 | 5.1% | 3.9% | 0.38 | 0.540 |
| 2 2 1 1 1 2 2 1 | 23 | 10 | 33 | 3.9% | 6.5% | 1.80 | 0.179 |
| 2 2 1 1 1 1 1 1 | 23 | 7.5 | 30.5 | 3.9% | 4.9% | 0.26 | 0.607 |
| 1 1 1 1 1 1 2 1 | 17 | 5 | 22 | 2.9% | 3.3% | 0.05 | 0.825 |
| 2 1 1 1 1 1 2 1 | 8 | 1 | 9 | 1.4% | 0.7% | 0.51 | 0.473 |
| 2 2 1 1 1 2 2 2 | 3 | 1 | 4 | 0.5% | 0.7% | 0.04 | 0.837 |
| 1 1 1 1 1 2 2 1 | 2 | 1 | 3 | 0.3% | 0.7% | 0.29 | 0.593 |
| 2 1 2 2 1 1 2 1 | 2 | 1 | 3 | 0.3% | 0.7% | 0.29 | 0.593 |
| TOTAL | 584 | 153.5 | 737.5 | | | 12.97 | 0.226 |
| Table 14C: Haplotype transmissions by AFBAC method: Aneither sib DR3/4@ subgroup of families | | | | | | | |

| | Transmitted (n=540) | | | AFBAC (n=121.5) | | | Chi-squared Statistic | p-value (df = 1) |
|---|---|---|---|---|---|---|---|---|
| SNP # | allele counts | | variant allele frequency | allele counts | | variant allele frequency | | |
| | wt | variant | | wt allele | variants | | | |
| 1 | 296 | 244 | 45.2% | 64.5 | 57 | 46.9% | 0.12 | 0.730 |
| 2 | 490 | 50 | 9.3% | 107.5 | 14 | 11.5% | 0.58 | 0.446 |
| 3 | 485 | 55 | 10.2% | 103.5 | 18 | 14.8% | 2.17 | 0.141 |
| 4 | 485 | 55 | 10.2% | 103.5 | 18 | 14.8% | 2.17 | 0.141 |
| 5 | 485 | 55 | 10.2% | 103.5 | 18 | 14.8% | 2.17 | 0.141 |
| 6 | 457 | 83 | 15.4% | 96.5 | 25 | 20.6% | 1.97 | 0.161 |
| 7 | 436 | 104 | 19.35 | 88.5 | 33 | 27.2% | 3.77 | 0.052 |
| 8 | 532 | 8 | 1.5% | 119.5 | 2 | 1.6% | 0.02 | 0.893 |
| | | | | | | | | |
| TOTAL | | | | | | | | |
| Table 15A. SNP by SNP allele transmissions by AFBAC method: Aeither/both sib DR3/4@ subgroup of familes | | | | | | | | |

| | Transmitted (n=588) | | | AFBAC (n=158.5) | | | Chi-squared Statistic | p-value (df = 1) |
|---|---|---|---|---|---|---|---|---|
| SNP # | allele counts | | variant allele frequency | allele counts | | variant allele frequency | | |
| | wt | variant | | wt allele | variant | | | |
| 1 | 320 | 268 | 45.6% | 77.5 | 81 | 51.1% | 1.53 | 0.216 |
| 2 | 539 | 49 | 8.3% | 139 | 19.5 | 12.3% | 2.36 | 0.124 |
| 3 | 529 | 59 | 10.0% | 132 | 26.5 | 16.7% | 5.50 | 0.019 |
| 4 | 529 | 59 | 10.0% | 132 | 26.5 | 16.7% | 5.50 | 0.019 |
| 5 | 534 | 54 | 9.2% | 136 | 22.5 | 14.2% | 3.41 | 0.065 |
| 6 | 502 | 86 | 14.6% | 121 | 37.5 | 23.7% | 7.38 | 0.007 |
| 7 | 475 | 113 | 19.2% | 115 | 43.5 | 27.4% | 5.10 | 0.024 |
| 8 | 584 | 4 | 0.7% | 157.5 | 1 | 0.6% | 0.00 | 0.946 |
| Table 15B. SNP by SNP allele transmissions by AFBAC method: Aneither sib DR3/4@ subgroup of familes | | | | | | | | |

| **haplotype** | **transmitted to affected** | **NOT transmitted to affected** | **TOTAL TRANSMISSIONS OBSERVED** | **McNemar chi- squared statistic** | **P-value (1df)** | **% TRANSMISSION** |
|---|---|---|---|---|---|---|
| 11111111 | 140 | 131 | 271 | 0.299 | 0.585 | 51.7% |
| 21111111 | 130 | 105 | 235 | 2.660 | 0.103 | 55.3% |
| 21222221 | 27 | 39 | 66 | 2.182 | 0.140 | 40.9% |
| 22111111 | 19 | 28 | 47 | 1.723 | 0.189 | 40.4% |
| 11222221 | 19 | 24 | 43 | 0.581 | 0.446 | 44.2% |
| 22111221 | 17 | 23 | 40 | 0.900 | 0.343 | 42.5% |
| 11111121 | 18 | 15 | 33 | 0.273 | 0.602 | 54.5% |
| 22111222 | 6 | 5 | 11 | 0.091 | 0.763 | 54.5 % |
| 21111121 | 1 | 5 | 6 | 2.667 | 0.102 | 16.7% |
| 11111221 | 1 | 3 | 4 | 1.000 | 0.317 | 25.0% |
| 11221221 | 2 | 0 | 2 | 2.000 | 0.157 | 100.0% |
| 21221121 | 1 | 1 | 2 | 0.000 | 1.000 | 50.0% |
| 21221221 | 0 | 2 | 2 | 2.000 | 0.157 | 0.0% |
| 11221111 | 0 | 1 | 1 | 1.000 | 0.317 | 0.0% |
| 11111222 | 1 | 0 | 1 | 1.000 | 0.317 | 100.0% |
| total | 382 | 382 | 764 | | | |
| Table 16A: TDT analysis of 8-loocus haplotypes to probands only. | | | | | | |

| **haplotype** | **transmitted to affected** | **NOT transmitted to affected** | **TOTAL TRANSMISSIONS OBSERVED** | **McNemar chi- squared statistic** | **P-value (1 df)** | **% TRANSMISSION** |
|---|---|---|---|---|---|---|
| 21222221 | 52 | 80 | 132 | 5.939 | 0.015 | 39.4% |
| 21111111 | 245 | 225 | 470 | 0.851 | 0.356 | 52.1% |
| 11111111 | 283 | 259 | 542 | 1.063 | 0.303 | 52.2% |
| 22111111 | 43 | 51 | 94 | 0.681 | 0.409 | 45.7% |
| 11221221 | 3 | 1 | 4 | 1.000 | 0.317 | 75.0% |
| 11222221 | 44 | 42 | 86 | 0.047 | 0.829 | 51.2% |
| 22111221 | 37 | 43 | 80 | 0.450 | 0.502 | 46.3 % |
| 11111121 | 36 | 30 | 66 | 0.545 | 0.460 | 54.5 % |
| 22111222 | 11 | 11 | 22 | 0.000 | 1.000 | 50.0% |
| 21111121 | 3 | 9 | 12 | 3.000 | 0.083 | 25.0% |
| 21221121 | 2 | 2 | 4 | 0.000 | 1.000 | 50.0% |
| 21221221 | 0 | 4 | 4 | 4.000 | 0.046 | 0.0% |
| 11111221 | 4 | 4 | 8 | 0.000 | 1.000 | 50.0% |
| 11221111 | 0 | 2 | 2 | 2.000 | 0.157 | 0.0% |
| 11111222 | 1 | 1 | 2 | 0.000 | 1.000 | 50.0% |
| Table 16B: TDT analysis of 8-loocus haplotypes to two primary affected children per family. | | | | | | |

| **haplotype** | **transmitted to affected** | **NOT transmitted to affected** | **TOTAL TRANSMISSIONS OBSERVED** | **McNemar chi- squared statistic** | **P-value (1df)** | **% TRANSMISSION** |
|---|---|---|---|---|---|---|
| 11111111 | 295 | 272 | 567 | 0.933 | 0.334 | 52.0% |
| 21111111 | 259 | 236 | 495 | 1.069 | 0.301 | 52.3% |
| 21222221 | 55 | 88 | 143 | 7.615 | 0.006 | 38.5% |
| 22111111 | 45 | 53 | 98 | 0.653 | 0.419 | 45.9% |
| 11222221 | 44 | 43 | 87 | 0.011 | 0.915 | 50.6% |
| 22111221 | 40 | 46 | 86 | 0.419 | 0.518 | 46.5% |
| 11111121 | 38 | 30 | 68 | 0.941 | 0.332 | 55.9% |
| 22111222 | 12 | 11 | 23 | 0.043 | 0.835 | 52.2% |
| 21111121 | 3 | 10 | 13 | 3.769 | 0.052 | 23.1% |
| 11111221 | 6 | 4 | 10 | 0.400 | 0.527 | 60.0% |
| 21221221 | 1 | 5 | 6 | 2.667 | 0.102 | 16.7% |
| 11221221 | 3 | 2 | 5 | 0.200 | 0.655 | 60.0% |
| 21221121 | 3 | 2 | 5 | 0.200 | 0.655 | 60.0% |
| II22I111 | 0 | 2 | 2 | 2.000 | 0.157 | 0.0% |
| 11111222 | 1 | 1 | 2 | 0.000 | 1.000 | 50.0% |
| TOTAL | 805 | 805 | 1610 | | | |
| Table 16c: TDT analysis of 8-loocus haplotypes to all affected children. | | | | | | |

| **haplotype** | **transmitted to affected** | **NOT transmitted to affected** | **TOTAL OBSERVED** | **McNemar chi-squared statistic** | **P-value (1dt)** | **% TRANSMISSION** |
|---|---|---|---|---|---|---|
| 11111111 | 133 | 121 | 254 | 0.567 | 0,451 | 52.45 |
| 21111111 | 105 | 101 | 206 | 0.078 | 0.780 | 51.0% |
| 21222221 | 30 | 36 | 66 | 0.545 | 0.460 | 45.5% |
| 22111111 | 22 | 26 | 48 | 0.333 | 0.564 | 45.8% |
| 11222221 | 19 | 19 | 38 | 0.000 | 1.000 | 50.0% |
| 22111221 | 16 | 22 | 38 | 0.947 | 0.330 | 42.1% |
| 22111222 | 14 | 14 | 28 | 0.000 | 1.000 | 50.0% |
| 22111222 | 8 | 6 | 14 | 0.286 | 0.593 | 57.1% |
| 21111121 | 1 | 5 | 6 | 2.667 | 0.102 | 16.7% |
| 11111221 | 2 | 0 | 2 | 2.000 | 0.157 | 100.0% |
| TOTAL | 350 | 350 | 700 | | | |
| Table 17A: analysis of 8-loocus haplotypes to two primary affected children per family: Aeither/both sib DR3/4@ subgroup. | | | | | | |

| **haplotype** | **transmitted to affected** | **NOT transmitted to affected** | **TRANSMISSIONS OBSERVED** | **McNemar chi-squared statistic** | **P-value (1 dt)** | **% TRANSMISSION %** |
|---|---|---|---|---|---|---|
| 111 I 1111 | 138 | 126 | 264 | 0.545 | 0.460 | 52.3% |
| 21111111 | 128 | 112 | 240 | 1.067 | 0.302 | 53.3% |
| 21222221 | 22 | 44 | 66 | 7.333 | 0.007 | 33.3% |
| 22111111 | 23 | 29 | 52 | 0.692 | 0.405 | 44.2% |
| 11222221 | 28 | 20 | 48 | 1.333 | 0.248 | 58.3% |
| 22111111 | 21 | 25 | 46 | 0.348 | 0.555 | 45.7% |
| 11111121 | 17 | 11 | 28 | 1.286 | 0.257 | 60.7% |
| 22111222 | 3 | 5 | 8 | 0.500 | 0.480 | 37.5% |
| 11111221 | 2 | 4 | 6 | 0.667 | 0.414 | 33.3% |
| 21111121 | 2 | 4 | 6 | 0.667 | 0.414 | 33.3% |
| 11221221 | 3 | 1 | 4 | 1.000 | 0.317 | 75.0% |
| 21221121 | 2 | 2 | 4 | 0.000 | 1.000 | 50.0% |
| 21221221 | 0 | 4 | 4 | 4.000 | 0.046 | 0.0% |
| 11221111 | 0 | 2 | 2 | 2.000 | 0.157 | 0.0% |
| 11111222 | 1 | 1 | 2 | 0.000 | 1.000 | 50.0% |
| TOTAL | 390 | 390 | 780 | | | |
| Table 17B: TDT analysis of 8-loocus haplotypes to two primary affected children per family: Aneither sib DR3/4@ subgroup.. | | | | | | |

| **SNP #** | **WT Allele Frequency in controls** | **WT allele frquency in diabetics** | **P-value by z-test** |
|---|---|---|---|
| 1 | 0.559 | 0.461 | 0.077 |
| 2 | 0.989 | 1.000 | 0.480 |
| 3 | 0.793 | 0.882 | 0.031 |
| 4 | 0.824 | 0.933 | 0.003 |
| 5 | 0.809 | 0.882 | 0.075 |
| 6 | 0.814 | 0.882 | 0.097 |
| 7 | 0.723 | 0.770 | 0.362 |
| 8 | 1.000 | 1.000 | 1.000 |
| n = | **188** | **178** | chromosomes |
| Table 18: Allele frequencies compared between Filipino controls and diabetics. | | | |

| | **frequency: controls** | **MLS frequency: Diabetics** | **O.R.** | **COUNT: control** | **COUNT: diabetics** | **Total count** | **exp control** | **exp diabetic** | **chi- square** | **P-value** |
|---|---|---|---|---|---|---|---|---|---|---|
| IL4R | | | | | | | | | | |
| 11111111 | 0.318 | 0.329 | 1.1 | 59.7 | 58.5 | 118.2 | 60.7 | 57.5 | 0.033 | 0.855 |
| 11111121 | 0.074 | 0.074 | 1.0 | 14.0 | 13.2 | 27.2 | 14.0 | 13.2 | 0.000 | 0.987 |
| 11222221 | 0.145 | 0.058 | 0.4 | 27.3 | 10.3 | 37.6 | 19.3 | 18.3 | 6.727 | 0.009 |
| 21111111 | 0.390 | 0.441 | 1.2 | 73.3 | 78.5 | 151.8 | 78.0 | 73.8 | 0.582 | 0.445 |
| 21212221 | 0.032 | 0.051 | 1.6 | 6.0 | 9.0 | 15.0 | 7.7 | 7.3 | 0.776 | 0.378 |
| n = | **188** | **178** | | | | **366** | | | | |
| Table 19: Computationally estimated haplotype frequencies compared between Filipino controls and diabetics. | | | | | | | | | | |

| **haplotype** | **frequency: controls** | **frequency: Diabetics** | **O.R.** | **control** | **diabetics** | **Total count** | **control** | **diabetic** | **square** | **P-value** |
|---|---|---|---|---|---|---|---|---|---|---|
| IL4R | | | | | | | | | | |
| x111111 1 | 0.707 | 0.770 | 1.4 | 133.0 | 137.0 | 270.0 | 138.7 | 131.3 | 0.480 | 0.489 |
| x1111121 | 0.074 | 0.112 | 1.6 | 14.0 | 20.0 | 34.0 | 17.5 | 16.5 | 1.413 | 0.235 |
| x122222 | 0.154 | 0.067 | 0.4 | 29.0 | 12.0 | 41.0 | 21.1 | 19.9 | 6.155 | 0.013 |
| x1212221 | 0.032 | 0.051 | 1.6 | 6.0 | 9.0 | 15.0 | 7.7 | 7.3 | 0.776 | 0.378 |
| x1222111 | 0.005 | 0.000 | 0.0 | 1.0 | 0.0 | 1.0 | 0.5 | 0.5 | 0.947 | 0.331 |
| x2111111 | 0.011 | 0.000 | 0.0 | 2.0 | 0.0 | 2.0 | 1.0 | 1.0 | 1.894 | 0.169 |
| x1221121 | 0.016 | 0.000 | 0.0 | 3.0 | 0.0 | 3.0 | 1.5 | 1.5 | 2.840 | 0.092 |
| | | | n = | **188.0** | **178.0** | **366** | | **overall** | **14.504** | **0.024** |
| Table 20: Observed haplotypes and frequencies compared between Filipino controls and diabetics.. | | | | | | | | | | |

## Claims

1. A method for determining an individual's risk for type 1 diabetes comprising, detecting the presence of an IDDM-associated IL4R allele in a nucleic acid sample of the individual, wherein the presence of said allele indicates the individual's risk for type 1 diabetes, and wherein the allele is detected by contacting the nucleic acid sample with one or more sequence-specific oligonucleotides capable of hybridizing to one or more polymorphisms listed in Table 2 and detecting the hybridized sequence- specific oligonucleotide.

2. The method of claim 1, wherein the risk for type 1 diabetes is an increased risk.

3. The method of claim 2, wherein the disease-associated allele is a predisposing allele.

4. The method of claim 1, wherein the risk for type 1 diabetes is a decreased risk.

5. The method of claim 4, wherein the disease-associated allele is a protective allele.

6. The method of claim 1, wherein the nucleic acid sample comprises DNA.

7. The method of claim 1, wherein the nucleic acid sample comprises RNA.

8. The method of claim 1, wherein the nucleic acid sample is amplified.

9. The method of claim 8, wherein the nucleic acid sample is amplified by a polymerase chain reaction.

10. The method of claim 1, wherein the allele is detected by amplification.

11. The method of claim 10, wherein the allele is detected by a polymerase chain reaction.

12. The method of claim 1, wherein the allele is detected by sequencing.

13. The method of claim 1, wherein the one or more sequence specific- oligonucleotides comprise 1, 2, 3, 4, 5, 6, 7 or 8 of the SNPs listed in Table 2.

14. The use of a kit for determining an individual's risk for type 1 diabetes wherein said kit comprises
(a) one or more sequence-specific oligonucleotides each individually comprising a sequence that is fully complementary to a sequence in an IDDM-associated IL4R allele listed in Table 2, wherein said sequence comprises one or more polymorphisms associated with said allele; and
(b) instructions to use the kit to determine the individual's risk for type 1 diabetes,

15. The use of claim 14, wherein said kit contains additional sequencing primers.

16. The use of claim 14, wherein one or more sequence-specific oligonucleotides of said kit are labeled.

17. The use of claim 16, whereby said kit includes a means to detect the labeL

18. The use of claim 14, wherein the one or more sequence-specific oligonucleotides of said kit are each individually complementary to a sequence in a predisposing IL4R allele.

19. The use of claim 14, wherein the one or more sequence-specific oligonucleotides of said kit are each individually complementary to a sequence in a protective IL4R allele.

20. The use of claim 14, wherein the one or more sequence-specific oligonucleotides of said kit comprise 1, 2, 3, 4, 5, 6, 7 or 8 of the SNPs listed in Table 2.

21. The in vitro use of one or more SNPs listed in Table 2 for determining an individual's risk for type 1 diabetes.

## Patentansprüche

1. verfahren zur Bestimmung der Gefährdung eines Individuums durch Diabetes Typ 1, bei dem man das Vorliegen eines mit IDDM assoziierten IL4R-Allels in einer Nukleinsäureprobe des Individuums nachweist, wobei das Vorliegen dieses Allels die Gefährdung des Individuums durch Diabetes Typ 1 anzeigt, und wobei das Allel **dadurch** nachgewiesen wird, daß man die Nukleinsäureprobe mit einem oder mehreren sequenzspezifischen Oligonukleotiden, das/die fähig ist/sind, mit einem oder mehreren in Tabelle 2 angeführten Polymorphismen zu hybridisieren, in Kontakt bringt und das hybridisierte sequenzspezifische Oligonukleotid nachweist.

2. Verfahren nach Anspruch 1, wobei es sich bei der Gefährdung durch Diabetes Typ 1 um eine erhöhte Gefährdung handelt.

3. Verfahren nach Anspruch 2, wobei das mit der Krankheit assoziierte Allel ein prädisponierendes Allel ist.

4. Verfahren nach Anspruch 1, wobei es sich bei der Gefährdung durch Diabetes Typ 1 um eine verringerte Gefährdung handelt.

5. Verfahren nach Anspruch 4, wobei das mit der Krankheit assoziierte Allel ein schützendes Allel ist.

6. Verfahren nach Anspruch 1, wobei die Nukleinsäureprobe DNA umfaßt.

7. Verfahren nach Anspruch 1, wobei die Nukleinsäureprobe RNA umfaßt.

8. Verfahren nach Anspruch 1, wobei die Nukleinsäureprobe amplifiziert wird.

9. Verfahren nach Anspruch 8, wobei die Nukleinsäureprobe durch eine Polymerasekettenreaktion amplifiziert wird.

10. Verfahren nach Anspruch 1, wobei das Allel durch Amplifikation nachgewiesen wird.

11. Verfahren nach Anspruch 10, wobei das Allel durch eine Polymerasekettenreaktion nachgewiesen wird.

12. Verfahren nach Anspruch 1, wobei das Allel durch Sequenzieren nachgewiesen wird.

13. Verfahren nach Anspruch 1, wobei das eine bzw. die mehreren sequenzspezifische(n) Oligonukleotid(e) 1, 2, 3, 4, 5, 6, 7 oder 8 der in Tabelle 2 angeführten SNPs umfaßt/umfassen.

14. Verwendung eines Kits zur Bestimmung der Gefährdung eines Individuums durch Diabetes Typ 1, wobei das Kit folgendes umfaßt:
(a) ein oder mehrere sequenzspezifische(s) Oligonukleotid(e), die jeweils einzeln eine Sequenz umfassen, die zu einer Sequenz in einem mit IDDM assoziierten in Tabelle 2 angeführten IL4R-Allel vollständig komplementär ist, wobei diese Sequenz einen oder mehrere mit dem Allel assoziierte Polymorphismen umfaßt, und
(b) Anweisungen zur Verwendung des Kits zur Bestimmung der Gefährdung des Individuums durch Diabetes Typ 1.

15. Verwendung nach Anspruch 14, wobei das Kit zusätzliche Sequenzier-Primer enthält.

16. Verwendung nach Anspruch 14, wobei eine oder mehrere sequenzspezifische Oligonukleotide des Kits markiert sind.

17. Verwendung nach Anspruch 16, wobei das Kit ein Mittel zum Nachweisen der Markierung beinhaltet.

18. Verwendung nach Anspruch 14, wobei das eine oder die mehreren sequenzspezifischen Oligonukeotide des Kits jeweils einzeln zu einer Sequenz in einem prädisponierenden IL4R-Allel komplementär sind.

19. Verwendung nach Anspruch 14, wobei das eine oder die mehreren sequenzspezifischen Oligonukeotide des Kits jeweils einzeln zu einer Sequenz in einem schützenden IL4R-Allel komplementär sind.

20. Verwendung nach Anspruch 14, wobei das eine oder die mehreren sequenzspezifischen Oligonukeotide des Kits 1, 2, 3, 4, 5, 6, 7 oder 8 der in Tabelle 2 angeführten SNPs umfassen.

21. In-vitro-Verwendung von einem oder mehreren in Tabelle 2 angeführten SNPs für die Bestimmung der Gefährdung eines Individuums durch Diabetes Typ 1.

## Revendications

1. Procédé de détermination d'un risque de diabète de type 1 d'un sujet comprenant, la détection de la présence d'un allèle de l'IL4R associé au DID dans un échantillon d'acide nucléique du sujet, dans lequel la présence dudit allèle indique le risque de diabète de type 1 du sujet, et dans lequel l'allèle est détecté en mettant en contact l'échantillon d'acide nucléique avec un ou plusieurs oligonucléotides spécifiques d'une séquence capables de s'hybrider à un ou plusieurs polymorphismes énuméré(s) dans le tableau 2 et la détection de l'oligonucléotide spécifique d'une séquence hybridé.

2. Procédé selon la revendication 1, dans lequel le risque de diabète de type 1 est un risque accru.

3. Procédé selon la revendication 2, dans lequel l'allèle associé à la maladie est un allèle de prédisposition.

4. Procédé selon la revendication 1, dans lequel le risque de diabète de type 1 est un risque diminué.

5. Procédé selon la revendication 4, dans lequel l'allèle associé à la maladie est un allèle protecteur.

6. Procédé selon la revendication 1, dans lequel l'échantillon d'acide nucléique comprend de l'ADN.

7. Procédé selon la revendication 1, dans lequel l'échantillon d'acide nucléique comprend de l'ARN.

8. Procédé selon la revendication 1, dans lequel l'échantillon d'acide nucléique est amplifié.

9. Procédé selon la revendication 8, dans lequel l'échantillon d'acide nucléique est amplifié par une réaction en chaîne par polymérase.

10. Procédé selon la revendication 1, dans lequel l'allèle est détecté par amplification.

11. Procédé selon la revendication 10, dans lequel l'allèle est détecté par une réaction en chaîne par polymérase.

12. Procédé selon la revendication 1, dans lequel l'allèle est détecté par séquençage.

13. Procédé selon la revendication 1, dans lequel le(s) un ou plusieurs oligonucléotides spécifiques d'une séquence comprennent 1, 2, 3, 4, 5, 6, 7 ou 8 des SNP énumérés dans le tableau 2.

14. Utilisation d'un kit pour déterminer un risque de diabète de type 1 d'un sujet, dans laquelle ledit kit comprend
(a) un ou plusieurs oligonucléotides spécifiques d'une séquence comprenant chacun, de manière individuelle, une séquence qui est entièrement complémentaire d'une séquence dans un allèle de l'IL4R associé au DID répertorié dans le tableau 2, dans laquelle ladite séquence comprend un ou plusieurs polymorphismes associé(s) audit allèle ; et
(b) des instructions pour utiliser le kit afin de déterminer le risque de diabète de type 1 d'un sujet.

15. Utilisation selon la revendication 14, dans laquelle ledit kit contient des amorces de séquençage supplémentaires.

16. Utilisation selon la revendication 14, dans laquelle un ou plusieurs oligonucléotides spécifiques d'une séquence dudit kit sont marqués.

17. Utilisation selon la revendication 16, moyennant quoi ledit kit inclut un moyen pour détecter la marque.

18. Utilisation selon la revendication 14, dans laquelle le(s) un ou plusieurs oligonucléotides spécifiques d'une séquence dudit kit sont chacun complémentaires, de manière individuelle, d'une séquence dans un allèle de prédisposition de l'IL4R.

19. Utilisation selon la revendication 14, dans laquelle le(s) un ou plusieurs oligonucléotides spécifiques d'une séquence dudit kit sont chacun complémentaires, de manière individuelle, d'une séquence dans un allèle protecteur de l'IL4R.

20. Utilisation selon la revendication 14, dans laquelle le(s) un ou plusieurs oligonucléotides spécifiques d'une séquence dudit kit comprennent 1, 2, 3, 4, 5, 6, 7 ou 8 des SNP énumérés dans le tableau 2.

21. Utilisation *in vitro* d'un ou plusieurs SNP énuméré(s) dans le tableau 2 pour la détermination d'un risque de diabète de type 1 d'un sujet.
